# EUROPÄISCHE PATENTSCHRIFT

(11) **EP 0 691 329 B1**
(45) Veröffentlichungstag und Bekanntmachung des Hinweises auf die Patenterteilung: **29.10.1997**
(21) Anmeldenummer: 95110218.5
(22) Anmeldetag: 30.06.1995
(51) Int. Cl.: C07C 403/20

(54) **Verbessertes Verfahren zur Herstellung von Polyencarbonylverbindungen mit einem hohen Gehalt an dem all-E-Isomeren, sowie von deren Acetalen oder Ketalen**
Process for the preparation of polyencarbonyl compounds with high content of the all E-isomer and their acetals or ketales
Procédé de préparation de composés polyènecarbonyl à forte teneur d'isomère all-E et leurs acétales ou kétales

(30) Priorität: 09.07.1994 DE 4424294; 28.02.1995 DE 19506999
(43) Veröffentlichungstag der Anmeldung: 10.01.1996
(73) Patentinhaber: BASF AKTIENGESELLSCHAFT, 67056 Ludwigshafen (DE)
(72) Erfinder: Dobler, Walter, Dr., D-69126 Heidelberg (DE); Krause, Wolfgang, Dr., D-68782 Brühl (DE); Paust, Joachim, Dr., D-67141 Neuhofen (DE); Wörz, Otto, Dr., D-67159 Friedelsheim (DE); Rheude, Udo, Dr., D-67166 Otterstadt (DE); Burst, Wolfram, D-68199 Mannheim (DE); Däuwel, Günter, D-67363 Lustadt (DE); Bertram, Armin, D-67227 Frankenthal (DE); Schulz, Bernhard, Dr., D-68723 Schwetzingen (DE); Wegner, Günter, Dr., D-67354 Römerberg (DE); Münster, Peter, Dr., D-67354 Römerberg (DE); Ernst, Hansgeorg, Dr., D-67346 Speyer (DE); Kochner, Arno, D-67165 Waldsee (DE); Etzrodt, Heinz, Dr., D-67434 Neustadt (DE)

(56) Entgegenhaltungen:
- EP-A- 0 010 208
- WO-A-92/00360

## Beschreibung

Die Erfindung betrifft ein verbessertes Verfahren zur Herstellung von Polyencarbonylverbindungen mit hohem all-E-Gehalt sowie von deren Acetalen oder Ketalen, insbesondere von Carotinoiden, wie β-Apo-8'-carotinsäureestern, Citranaxanthin und Neurosporaxanthinestern.

Unter Polyenen versteht man ganz allgemein ungesättigte aliphatische Kohlenwasserstoffe mit drei oder mehr konjugierten Doppelbindungen im Molekül, d.h. Verbindungen mit zahlreichen alternierenden Einfach- und Doppelbindungen. Als Polyencarbonylverbindungen definieren wir Polyene, die eine Carbonylgruppe in Konjugation zu der Polyenkette aufweisen.

Viele Polyene und Polyencarbonylverbindungen haben als biologisch medizinische Wirkstoffe oder als Lebensmittelfarbstoffe und Futtermittelzusatzstoffe Interesse erlangt.

Entsprechend ihrer Bedeutung sind zahlreiche Methoden zur Herstellung von Polyenen und Polyencarbonylverbindungen entwickelt worden (Übersicht vgl. O. Isler in "Carotenoids", Birkhauser-Verlag, 1971).

Bekannte Methoden zur Herstellung von Polyenen sind beispielsweise die sogenannte Horner-Emmons-Reaktion, d.h. die Verknüpfung von Aldehyden und Ketonen mit entsprechenden Phosphonsäureestern in Gegenwart von Basen (vgl. Houben-Weyl "Methoden der Organischen Chemie" 5/1d (1972) Seiten 127 bis 129 oder Pure & Appl. Chem. Vol. 63, No. 1 (1991) Seiten 45-58) und die Aldolkondensation, d.h. die basenkatalysierte Verknüpfung von Ketonen und/oder Aldehyden mit CH-aciden Verbindungen zu β-Hydroxy-carbonylverbindungen und anschließende Abspaltung von Wasser unter Ausbildung eines Systems konjugierter Doppelbindungen (vgl. Houben-Weyl 5/1d (1972) Seiten 142-144 oder J.Org.Chem. 30 (1965) Seite 2481). Solche C-C-Verknüpfungen unter Bildung einer Doppelbindung können zu Doppelbindungen mit Z- oder E-Konfiguration führen. Bei den bisher bekannten Verfahren wurden im allgemeinen Produkte erhalten, die nur in unbefriedigendem Maße aus all-E-Isomeren bestanden. Da die meisten der begehrten natürlichen Polyencarbonylverbindungen all-E-Konfiguration aufweisen, ist es zweckmäßig von all-E-Polyenbausteinen auszugehen und die C-C-Verknüpfung unter Reaktionsbedingungen durchzuführen, die bevorzugt zu einer Doppelbindung mit E-Konfiguration führen sowie eine Isomerisierung der Polyenbausteine zu Z-Isomeren möglichst vollständig zu verhindern.

Es war daher die Aufgabe der Erfindung Reaktionsbedingungen zu finden, unter denen die Verknüpfung von Polyenbausteinen zu Polyencarbonylverbindungen bevorzugt unter Bildung einer Doppelbindung mit E-Konfiguration und unter Erhalt der E-Konfiguration der Doppelbindungen im Ausgangsprodukt verläuft.

Es wurde nun überraschend gefunden, daß man bei den genannten Olefinverknüpfungsreaktionen bevorzugt eine Verknüpfung in E-Konfiguration unter Beibehaltung der E-Konfiguration in den Ausgangsverbindungen erhält, wenn man die Umsetzung in Gegenwart von Sauerstoff, Stickstoffmonoxid, bestimmten stabilen Radikalen bzw. von Radikalfängern und/oder in Gegenwart von bestimmten Chinonen, Chinonderivaten oder Coenzym-Q10-Hydroxychinon, durchführt.

Gegenstand der Erfindung ist dementsprechend ein Verfahren zur Herstellung von Polyencarbonylverbindungen mit hohem all-E-Gehalt oder deren Acetalen bzw. Ketalen durch eine Horner-Emmons-Reaktion oder eine Aldolkondensation einer geeigneten Carbonylverbindung mit einem geeigneten Dialkylphosphonat bzw. einer geeigneten CH-aciden Verbindung, das dadurch gekennzeichnet ist, daß man die Umsetzung zum Zwecke der bevorzugten Bildung einer Doppelbindung mit E-Konfiguration und um die E-Konfiguration der Doppelbindungen in den Ausgangsverbindungen möglichst vollständig zu erhalten in Gegenwart von Sauerstoff oder einem Sauerstoff-Inertgas-Gemisch, oder in Gegenwart von Stickstoffmonoxid oder einem Stickstoffmonoxid-Inertgas-Gemisch und/oder in Gegenwart von einem stabilen Radikal der allgemeinen Formel I 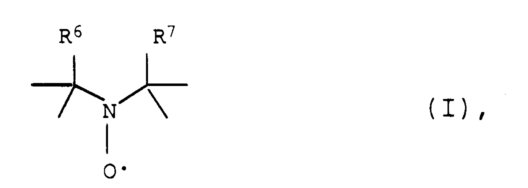 in der R⁶ und R⁷ für eine C₁- bis C₄-Alkylgruppe stehen oder aber
- R⁶ und R⁷: zusammen für eine Ethylengruppe, Propylengruppe, Vinylengruppe oder Propenylengruppe stehen, die durch Alkyl-, Aryl-, Hydroxyl-, Alkyloxy-, Silyloxy-, Oxo-, Amino-, Mercapto-, Alkylmercapto-, Cyano-, Carboxyl- oder Aminocarbonyl(Carbamoyl-), Heteroaryl- oder Alkylcarbonyloxygruppen substituiert sein können;
und/oder in Gegenwart eines stabilen Radikals der allgemeinen Formel II 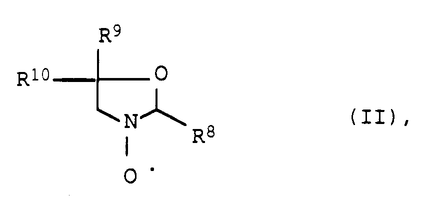 in der R⁸, R⁹ und R¹⁰ die oben für R⁶ und R⁷ angegebene Bedeutung haben können;
und/oder in Gegenwart von dem stabilen Radikal 2,2-Diphenyl-1-picrylhydrazyl, oder einem Wasserstoffperoxid-Harnstoffaddukt und/oder in Gegenwart von Chinonen, Chinonderivaten oder Coenzym-Q10-Hydrochinon durchführt, wobei man den Sauerstoff, das Stickstoffmonoxid, die genannten stabilen Radikale bzw. das Wasserstoffperoxid-Harnstoffaddukt, die Chinone, Chinonderivate oder Coenzym-Q10-hydrochinon in Mengen von 0,3 bis 10 Mol-%, vorzugsweise 0,5 - 6 Mol-%, insbesondere 1,0 bis 5 Mol-%, bezogen auf die eingesetzte Carbonylverbindung, verwendet.

Nach dem erfindungsgemäßen Verfahren können beispielsweise Polyencarbonylverbindungen der allgemeinen Formel III, deren Acetale oder Ketale hergestellt werden.

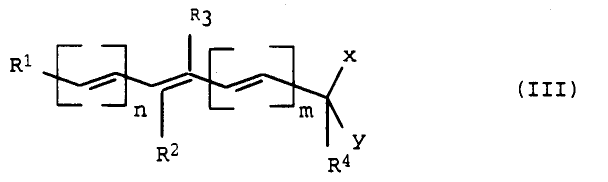

Hierin stehen R¹ bis R⁴ für Wasserstoff oder organische Reste; n für eine ganze Zahl von 0 bis 20, insbesondere 3 - 10; m für eine ganze Zahl von 0 bis 20, insbesondere 0 bis 10, wobei m + n mindestens 2 ist; X und Y für eine C₁- bis C₄-Alkoxygruppe insbesondere eine Methyloxy- oder Ethyloxygruppe stehen oder aber X und Y zusammen für Sauerstoff oder einen ggf. durch eine oder mehrere Methylgruppen substituierten Rest -O-CH₂-CH₂-O-, -O-CH₂-C(CH₃)₂-CH₂-O-, -O-CH=CH-O- oder -O-CH₂-CH₂-CH₂-O- stehen. Die Wasserstoffatome in den Klammern können teilweise durch organische Reste, vorzugsweise Alkylgruppen, insbesondere eine Methylgruppe, substituiert sein.

Als geeignete Polyenbausteine kommen dementsprechend beispielsweise Carbonylverbindungen der allgemeinen Formel IV 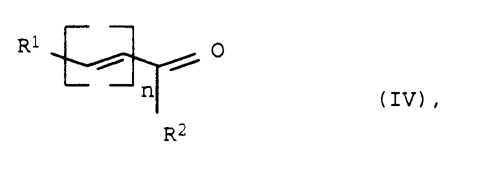 in der R¹ und R² für Wasserstoff oder organische Reste stehen, n für eine ganze Zahl von 0 bis 20, insbesondere 3 bis 10 steht und die Wasserstoffatome an den Doppelbindungen innerhalb der Klammer auch durch organische Reste ersetzt sein können.

Die als Ausgangsverbindungen verwendeten Carbonylverbindungen der allgemeinen Formel IV sind im allgemeinen bekannte Verbindungen. Geeignete Polyenbausteine sind z.B. Verbindungen der Formel IV, in der R¹ und R² für Wasserstoff, Alkyl-, Alkyloxy-, Cycloalkyl-, Cycloalkenyl- oder Phenylgruppen stehen, wobei die Cycloalkyl-, Cycloalkenyl- und Phenylgruppen noch substituiert sein können, beispielsweise durch Alkyl-, Hydroxy-, Oxo-, Amino-, Carboxyl-, Carbamoyl-, Alkylcarbonyloxy- oder Cyanogruppen oder auch durch Halogen, stehen.

R¹ steht bevorzugt für folgende Cycloalkenylgruppen 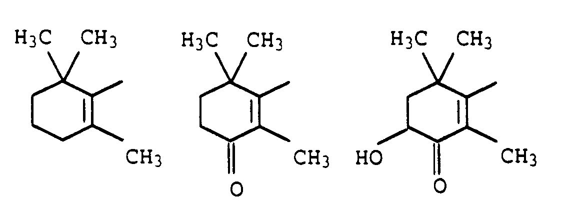 oder aber für eine Formylgruppe
während R² bevorzugt für Wasserstoff oder Alkyl steht.

Die Anzahl der Doppelbindungen in den Verbindungen der Formel IV (genannt n) beträgt vorzugsweise 2 bis 15, insbesondere 3 bis 10. Als organische Reste können die Doppelbindungen innerhalb der Klammer insbesondere C₁- bis C₄-Alkylgruppen, vorzugsweise Methylgruppen tragen. Als geeignete Carbonylverbindungen seien beispielsweise genannt: β-Apo-12'-carotinal (C₂₅-Aldehyd), β-Apo-8'-carotinal (C₃₀-Aldehyd), Retinal (C₂₀-Aldehyd), 2,7-Dimethyl-2,4,6-octatrien-dial (C₁₀-Dialdehyd), Crocetindialdehyd (C₂₀-Dialdehyd) und β-Apo-4'-carotinal (C₃₅-Aldehyd).

Bei Einsatz von Dialdehyden können durch Umsatz mit 2 Molen der Verbindungen der Formel V auch Verbindungen der allgemeinen Formel IX 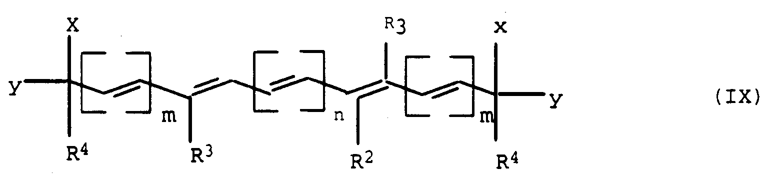 gebildet werden.

Zur Herstellung von Polyencarbonylverbindungen der Formel III mit bevorzugt all-E-Konfiguration müssen auch die Verbindungen der Formel IV bevorzugt all-E-Konfiguration aufweisen.

Auch die Carbonylverbindungen sowie deren Acetale oder Ketale der allgemeinen Formel V sind im allgemeinen bekannte Verbindungen. Geeignet sind vor allem Verbindungen der Formel V 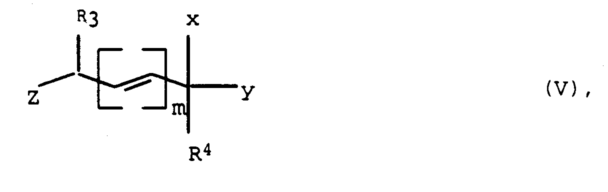 in der R³ und R⁴ für organische Reste stehen,
m für eine ganze Zahl von 0 bis 20, vorzugsweise 0 bis 10 steht, X und Y für eine C₁- bis C₄-Alkylgruppe, insbesondere eine Methylgruppe oder Ethylgruppe stehen
oder aber X und Y zusammen für Sauerstoff oder einen ggf. durch eine oder mehrere Methylgruppen substituierten Rest -O-CH₂-CH₂-O-, -O-CH₂-C(CH₃)₂-CH₂-O-, -O-CH=CH-O- oder -O-CH₂-CH₂-CH₂-O- stehen und Z für Wasserstoff oder eine Dialkylphosphonogruppe, insbesondere für -PO(OCH₃)₂ oder -PO(OC₂H₅)₂ steht. Die Anzahl der Doppelbindungen (genannt m) kann O sein (wie im Aceton), aber auch bis zu 20, vorzugsweise 0 bis 10. R³ steht für Wasserstoff, eine Alkyl-, Alkyloxy-, Cycloalkyl-, Cycloalkenyl- oder Phenylgruppe, die auch durch andere Reste, wie C₁- bis C₄-Alkyl-, Hydroxyl-, Alkyloxy-, Silyloxy-, Oxo-, Amino-, Cyano-, Carboxyl-, Carbamoyl- oder Alkylcarbonyloxygruppen substituiert sein können.

R⁴ steht - wie auch R³ - vorzugsweise für Wasserstoff, C₁- bis C₄-Alkyl- oder C₁- bis C₄-Alkyloxygruppen.

Bei der Durchführung einer Horner-Emmons-Reaktion können Polyenverbindungen der allgemeinen Formel III aber auch dadurch erhalten werden, daß man Polyenbausteine verwendet, die die reagierenden Gruppen jeweils am anderen Baustein enthalten, d.h. Verbindungen der allgemeinen Formel IV, in der anstelle der Gruppe 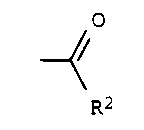 eine Gruppe 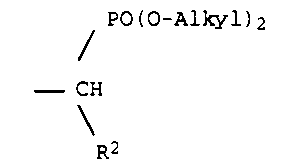 enthalten ist, werden umgesetzt mit Verbindungen der allgemeinen Formel V, in der anstelle der Gruppe 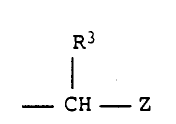 die Gruppe 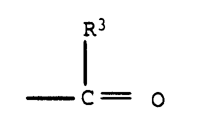 enthalten ist.

Als Carbonylverbindungen der allgemeinen Formel V seien beispielsweise genannt:
Aceton, 4-Dimethylphosphono-2-methyl-2-butensäureethylester, 4-Diethylphosphono-2-methyl-2-butensäureethylester, 4-Diethylphosphono-3-methyl-2-butensäureester, 2-Diethylphosphono-essigsäuremethylester, 2-Diethylphosphono-essigsäureethylester, 4-Diethylphosphono-2- oder 4-Diethylphosphono-3-methyl-2-butenalacetale.

Zur Herstellung von Polyencarbonylverbindungen der Formel III mit überwiegend all-E-Konfiguration müssen auch die höheren Carbonylverbindungen der Formel IV bzw. V überwiegend all-E-Konfiguration aufweisen. Bei Einsatz von Verbindungen mit nur einer Doppelbindung treten bei der erfindungsgemäßen Umsetzung in alkalischem Milieu zusätzlich Isomerisierungen zu Gunsten einer all-E-Konfiguration auf, so daß beispielsweise bei der Horner-Emmons-Reaktion von C₂₅-Aldehyd oder C₃₀-Aldehyd mit 4-Dialkylphosphono-2-methyl-2-butensäurealkylestern der C₅-Baustein in einem E/Z-Verhältnis von 2 : 1 bis 100 : 1 eingesetzt werden kann und trotzdem all-E-Selektivitäten von 85 bis 95 % erhalten werden.

Den Sauerstoff verwendet man aus Sicherheitsgründen im allgemeinen nicht in reiner Form, sondern bevorzugt als Sauerstoff-Inertgas-Gemisch. Als Inertgas bietet sich Stickstoff an. Vorzugsweise verwendet man Sauerstoff im Gemisch mit Stickstoff im Verhältnis von N₂ zu O₂ von 1 zu 1 bis 100:1, vorzugsweise etwa 95 zu 5 Vol-%, (sogenannte "Magerluft") an. Den Sauerstoff bzw. das Sauerstoff enthaltende Gasgemisch kann man über das gerührte Reaktionsgemisch leiten oder aber in das Reaktionsgemisch einleiten. Die Wirkung des Sauerstoffs ist abhängig von seiner Löslichkeit, bzw. von seinem Partialdruck im Reaktionsgemisch. Aus diesem Grunde ist es auch vorteilhafter, den Sauerstoff in das Reaktionsgemisch einzuleiten statt nur darüber.

Es war sehr überraschend, daß der Sauerstoff bei dem erfindungsgemäßen Verfahren einen so vorteilhaften Einfluß ausübt. Im allgemeinen wird nämlich beim Umgang mit Polyenen zum Arbeiten unter Sauerstoffausschluß geraten (vgl. Houben-Weyl 5/1d Seite 13), da die Polyene vielfach oxidationsempfindliche Doppelbindungen enthalten.

Überraschenderweise übt auch Stickstoffmonoxid bzw. ein Stickstoffmonoxid-Inertgas-Gemisch bei Aldol- oder Horner-Emmons-Reaktionen einen katalytischen Effekt zugunsten der Bildung einer Doppelbindung mit E-Konfiguration aus. Auch das Stickstoffmonoxid kann man - vorzugsweise im Gemisch mit einem Inertgas wie N₂ - kontinuierlich in das Reaktionsgemisch einleiten. Bei diskontinuierlicher Fahrweise genügt es, wenn man das benötigte Stickstoffmonoxid vor der Reaktion in das Reaktionsgefäß einleitet und dafür sorgt, daß es während der Umsetzung nicht entweichen kann.

Der Wirkungsmechanismus des erfindungsgemäßen Verfahrens ist noch nicht bekannt. Als Katalysatoren einsetzbar sind neben Sauerstoff und NO stabile Radikale, bei denen ein Stickstoffradikal oder ein N-Oxyl im Molekül vorhanden sind. Bei Anwendung von stabilen Radikalen kann die Umsetzung unter Inertgasschutz durchgeführt werden. Bevorzugt verwendet man als stabile Radikale der allgemeinen Formel I Di-tert-butyl-amin-N-oxyl der Formel Ia; 2,2,6,6-Tetramethyl-piperidin-N-oxyle der Formel Ib 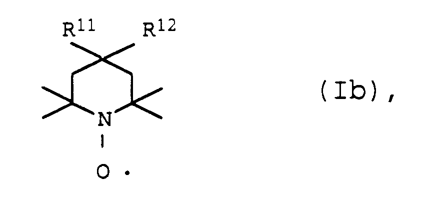 in der R¹¹ für Wasserstoff
und R¹² für Wasserstoff, eine Hydroxyl-, Alkoxy-, Acetoxy-, Amino-, Cyano- oder eine Carboxylgruppe stehen,
oder aber R¹¹ und R¹² zusammen für eine Oxogruppe stehen;
2,2,5,5-Tetramethyl-pyrrolidin-N-oxyle der Formel Ic 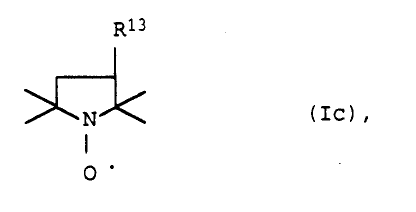 in der R¹³ für Wasserstoff oder eine der oben für R¹² angegebenen Gruppen steht oder
2,5-Dihydro-2,2,5,5-tetramethyl-1H-pyrrol-N-oxyle der Formel Id 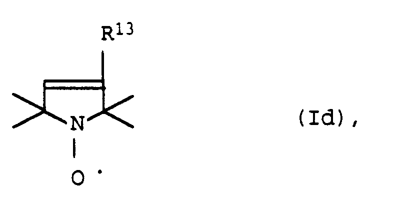 in der R¹³ für Wasserstoff oder eine der oben für R¹² angegebenen Gruppen steht.

Als besonders geeignete stabile Radikale seien z,B, genannt: 2,2,6,6-Tetramethyl-piperidin-N-oxyl (TEMPO), 4-Hydroxy-2,2,6,6-tetramethyl-piperidin-N-oxyl (4-OH-TEMPO), oder Ester mit ein- oder zweibasischen Carbonsäuren hiervon, wie das kommerziell erhältliche Bis-(1-oxyl-2,2,6,6-tetramethyl-piperidin-4-yl)-sebacat, 4-Oxo-2,2,6,6-tetramethyl-piperidin-N-oxyl, 3-Carboxy-2,2,5,5-tetramethyl-pyrrolidin-N-oxyl, 3-Aminocarbonyl-2,5-dihydro-2,2,5,5-tetramethyl-1H-pyrrol-N-oxyl, Di-tert.-butylamin-N-oxyl oder 2,2-Diphenyl-1-picrylhydrazyl.

Auch die stabilen Radikale verwendet man im allgemeinen in Mengen von 0,3 bis 10 Mol-%, vorzugsweise 0,5 bis 6 Mol-%, insbesondere 1 bis 5 Mol-%, bezogen auf die Polyencarbonylverbindung der Formel IV oder V.

Die Reaktionszeiten sind je nach Art der Reaktion und der Reaktionspartner recht unterschiedlich. Beispielsweise betragen sie bei der Umsetzung von β-Apo-12'-carotinal (C₂₅-Aldehyd) oder β-Apo-8'-carotinal (C₃₀-Aldehyd) mit 4-Diethylphosphono-2-methyl-2E-butensäureethylester, d.h. bei einer Horner-Emmons-Reaktion mit einem C₅-phosphonat im allgemeinen 1 bis 20 Stunden, vorzugsweise 2 bis 5 Stunden. Bei Verwendung der stabilen Radikale in Mengen von weniger als 1 Mol-% jedoch werden die Reaktionszeiten sehr viel länger, wenn man optimale all-E-Selektivitäten erreichen möchte.

Geeignete Katalysatoren zur Ausführung von all-E-selektiven Horner-Emmons-Reaktionen sind ganz allgemein Verbindungen, die selbst als Oxidationsmittel wirken und leicht in die reduzierte Form bzw. in die Hydro-Derivate überführbar sind, aber keine Oxidationen oder Additionen am Polyenbaustein verursachen. Dieses gilt auch für Chinone und Chinonimine. Chinone und Chinonimine sind allgemein bekannte, häufig physiologisch aktive Verbindungen. Die biologisch aktiven Chinone leiten sich im wesentlichen von 3 Chinonkernen ab, dem 1,4-Naphthochinon, dem methylsubstituierten 1,4-Benzochinon und dem methyl- und methoxysubstituierten 1,4-Benzochinon. Sie können eine Phytyl- oder abgeleitete Phytylylgruppe oder eine Multiisoprenylgruppe enthalten. Genannt seien sogenannte Tocochinone, wie Vitamin-E₂ (50), die sich von 2,3,5-Trimethyl-1,4-benzochinon ableiten; sogenannte Plastochinone, die sich von 2,3-Dimethyl-1,4-benzochinon ableiten; sogenannte Ubichinone, wie Coenzym Q10, Coenzym Q6 oder Coenzym Q0, die sich von 2,3-Dimethoxy-5-methyl- 1,4-benzochinon ableiten, sogenannte Menachinone, wie Vitamin K₂ (50) und Vitamin K₂ (30), die sich von 2-Methyl-naphtho-1,4-chinon ableiten und sogenannte Phyllochinone, wie Vitamin K₁, die sich von einem Phytyl-sübstituierten 2-Methyl-naphtho-1,4-chinon ableiten. Bezüglich näherer Angaben über die Struktur biologisch aktiver Chinone verweisen wir auf das "Handbuch zur Anwendung der Nomenklatur organischchemischer Verbindungen" von W. Liebscher, Akademie-Verlag, Berlin 1979, Seiten 838 bis 847.

Besonders geeignet sind Chinone oder Chinonimine der allgemeinen Formeln X oder XI 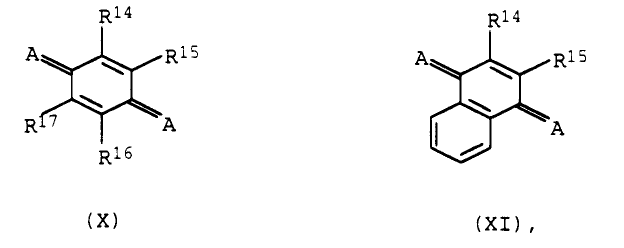 in denen A für =0, =NH oder =N-R⁵ steht, worin R⁵ für eine Alkylgruppe steht oder aber R⁵ zusammen mit einem der benachbarten Reste R¹⁴ bis R¹⁷ einen Alkylenrest oder Alkenylenrest mit 2 oder 3 C-Atomen bildet, der durch Halogen, eine C₁- bis C₄-Alkylgruppen oder C₁- bis C₄-Alkoxygruppen substituiert sein kann; R¹⁴ bis R¹⁷ für Wasserstoff, Halogen oder eine Alkyl-, Alkenyl-, Cycloalkyl-, Alkoxy-, Alkoxycarbonyl-, Cyano-, Acyloxy-, Aryl- oder Heteroarylgruppe stehen, wobei R¹⁵ zusätzlich für eine Phytylgruppe, eine von der Phytylgruppe abgeleitete Gruppe, eine Isoprenylgruppe oder eine Multiisoprenylgruppe stehen kann.

Für das erfindungsgemäße Verfahren sind sowohl elektronenreiche Chinone oder Chinonderivate der Formeln X oder XI, wie Coenzym Q0, Coenzym Q10 oder Tocochinone, als auch elektronendefizitäre Chinone oder Chinonderivate, wie Tetrachlor-1,4-benzochinon oder Dichlor-dicyano-1,4-benzochinone geeignet.

Es war besonders überraschend zu finden, daß auch starke Oxidantien, wie Chloranil (Tetrachlor-1,4-benzochinon) oder Dichloro-dicyano-benzochinone (DDQ) eine hohe Wirksamkeit und Selektivität zeigen, d.h. all-E-selektierend wirken, ohne Nebenreaktionen zu verursachen.

Mit Vorteil verwendet man erfindungsgemäß Chinolone der allgemeinen Formel Xa 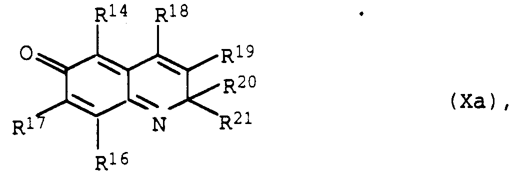 in der R¹⁴, R¹⁶ und R¹⁷ die oben angegebene Bedeutung haben und R¹⁸ bis R²¹ für C₁- bis C₄-Alkyl- oder Alkoxygruppen oder Halogen stehen,
insbesondere das Chinolon der Formel Xb 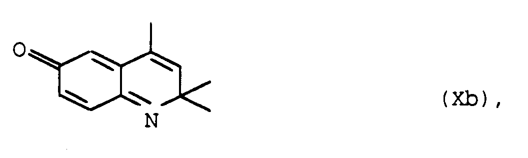 das sich von dem bekannten antioxidierend wirkenden Stabilisator Ethoxyquin ableiten läßt und als Metabolit von Ethoxyquin bekannt ist (vgl. Xenobiotica 9 (1979), Seiten 649 bis 57). Ethoxyquin selbst bewirkt nur eine mäßige Verbesserung der all-E-Selektivität, während das Chinolon Xb zu den aktivsten Katalysatoren zu zählen ist.

Weitere aktive Derivate des Ethoxyquins sind die Dimeren der Formeln XIIa und XIIb 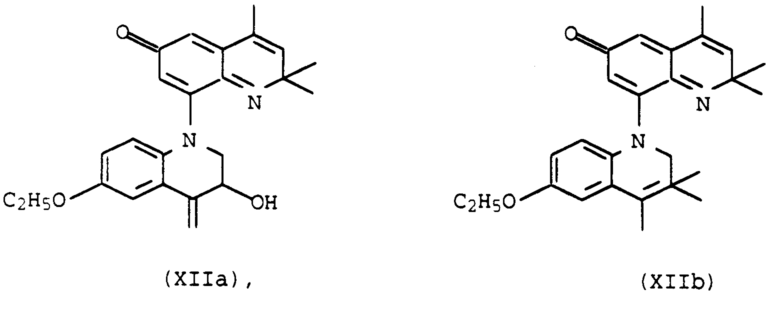 sowie N-Oxide der allgemeinen Formel XIII 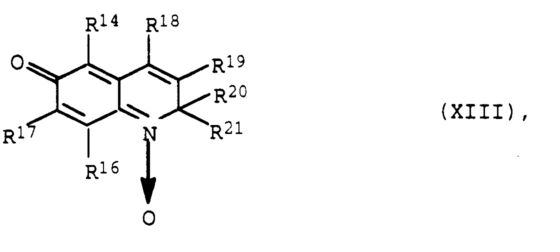 in der R¹⁴ und R¹⁶ bis R²¹ die oben angegebene Bedeutung haben, insbesondere aber für C₁- bis C₄-Alkyl- oder Alkoxygruppen oder Halogen stehen.

Neben den para-Chinonen können aber auch ortho-Chinone erfindungsgemäß eingesetzt werden, da auch diese recht hohe Redoxpotentiale aufweisen. Bezüglich weiterer Einzelheiten über ortho-Chinone verweisen wir auf Houben-Weyl Bd. 7/3b, Seiten 3 bis 6.

Überraschenderweise zeigen auch einzelne Hydrochinone, wie das Coenzym-Q10-Hydrochinon, eine höhere Wirksamkeit. Möglicherweise sind es solche Hydrochinone, die besonders leicht in Chinone überführbar sind.

Als geeignete chinoide Verbindungen seien insbesondere genannt: 1,4-Benzochinon, Dimethyl-1,4-benzochinon, Trimethyl-1,4-benzochinon, Naphthochinon, Tetrachlor-1,4-benzochinon, Tocochinonacetat, Phytyl-trimethyl-1,4-benzochinon, Chinone der Vitamin-K-Reihe und Coenzym-Q10, Coenzym-Q0 oder 2,2,4-Trimethyl-6(H)-chinolon.

Auch die Chinone und Chinonimine werden im allgemeinen in Mengen von 0,3 bis 10 Mol-%, vorzugsweise 0,5 bis 6 Mol-%, insbesondere 1,0 bis 5 Mol-%, bezogen auf die eingesetzte Carbonylverbindung eingesetzt.

Die Reaktionszeiten sind je nach Art der Reaktion und der Reaktionspartner recht unterschiedlich. Beispielsweise betragen sie bei der Umsetzung von β-Apo-12'-carotinal (C₂₅-Aldehyd) oder β-Apo-8'-carotinal (C₃₀-Aldehyd) mit 4-Diethylphosphono-2-methyl-2E-butensäureethylester, d.h. bei einer Horner-Emmons-Reaktion mit einem C₅-phosphonat im allgemeinen 1 bis 20 Stunden, vorzugsweise 2 bis 5 Stunden. Bei Verwendung der Chinone oder Chinonderivate in Mengen von weniger als 0,5 Mol-% jedoch werden die Reaktionszeiten länger, wenn man optimale all-E-Selektivitäten erreichen möchte.

Für die Durchführung von Horner-Emmons-Reaktion sind sowohl Sauerstoff als auch NO, stabile Radikale, Chinone, Chinonderivate und Coenzym-Q10-hydrochinon als Katalysatoren geeignet. Für die Durchführung von Aldolkondensationen werden dagegen O₂ und NO bevorzugt.

Zur Durchführung der erfindungsgemäßen Umsetzung geht man im allgemeinen so vor, wie es für die Aldolkondensation bzw. die Horner-Emmons-Reaktion bekannt und üblich ist, nur daß man während der Umsetzung Sauerstoff oder ein Sauerstoff-Inertgas-Gemisch oder Stickstoffmonoxid bzw. ein Stickstoffmonoxid-Inertgasgemisch über das Reaktionsgemisch oder in das Reaktionsgemisch leitet und/oder die Umsetzung in Gegenwart der beanspruchten stabilen Radikale, Chinone, Chinonderivate oder Coenzym-Q10-hydrochinon durchführt. Bei Verwendung von stabilen Radikalen, Chinonen, Chinonderivaten oder Coenzym-Q10-hydrochinon wird die Umsetzung im allgemeinen unter Inertgasschutz durchgeführt.

Unter einer Aldolkondensation verstehen wir im Rahmen der vorliegenden Erfindung die Verknüpfung der Carbonylverbindung der Formel IV mit einer Verbindung der allgemeinen Formel V, in der Z für Wasserstoff steht, als CH-aktiver Verbindung in Gegenwart einer starken Base. Als starke Basen können insbesondere Alkalihydroxyde, Alkalialkoxide, Alkalihydride oder Alkali-hexaalkylbissilazide verwendet werden. In Einzelfällen genügt auch die Verwendung einer schwachen Base, wie Soda.

Die Umsetzung wird im allgemeinen in einem Lösungsmittel durchgeführt, gelingt aber auch in einigen Fällen ohne Lösungsmittel. In vielen Fällen kann auch ein Überschuß der stabileren Ausgangsverbindung als Lösungsmittel dienen. Als geeignete Lösungsmittel kommen in Betracht: acyclische, cyclische oder aromatische Kohlenwasserstoffe oder Halogenkohlenwasserstoffe, wie Dichlorethylen, Alkanole, wie Methanol, Ethanol oder iso-Propanol oder Gemische der genannten Lösungsmittel oder aber polare aprotische Lösungsmittel, wie Tetrahydrofuran, Dimethylformamid oder Diethoxyethan.

Zur Durchführung der Reaktion geht man im allgemeinen so vor, daß man zu der Mischung der Ausgangsverbindungen in einem Lösungsmittel in Gegenwart von 0₂, NO oder einem der beanspruchten Katalysatoren langsam eine Lösung etwa äquimolarer Mengen der Base zufügt und das Reaktionsgemisch in an sich bekannter Weise aufarbeitet. Es ist aber in vielen Fällen auch möglich, den einen Reaktanden mit der Base vorzulegen und den zweiten Reaktanden langsam zuzufügen.

Die Reaktionstemperaturen sollten bei der Umsetzung etwa bei -70 bis 100°C, vorzugsweise -20 bis 70°C liegen.

Die Reaktionszeiten betragen je nach Art der Reaktanden, den Reaktionstemperaturen und der Menge des Katalysators 0,5 bis 24 Stunden, vorzugsweise 1 bis 10 Stunden. Bezüglich näherer Einzelheiten über Aldolkondensationen verweisen wir auf Hoüben-Weyl, Bd. 5/1 (1972) Seite 142 bis 144.

Unter einer Horner-Emmons-Reaktion verstehen wir im Rahmen der vorliegenden Erfindung die Umsetzung einer Carbonylverbindung der allgemeinen Formel IV mit einer Verbindung der allgemeinen Formel V, in der Z für eine Dialkylphosphono-Gruppe steht, in Gegenwart einer Base.

Als geeignete starke Basen sind auch hier alle Alkalihydroxide, Alkalialkoxide, Alkalihydride und Alkali-hexaalkylbissilazide sowie LiNH₂ und NaNH₂, im Einzelfall auch Alkalicarbonate, zu nennen. Die Umsetzung wird im allgemeinen in einem Lösungsmittel durchgeführt, gelingt aber in einigen Fällen auch ohne Lösungsmittel. Als Lösungsmittel können im allgemeinen die oben für die Aldolkondensation genannten Lösungsmittel eingesetzt werden. Mit besonderem Vorteil arbeitet man in Kohlenwasserstoffen oder in Gemischen aus Kohlenwasserstoffen und Alkanolen.

Auch hierbei geht man im allgemeinen so vor, daß man zu der Mischung aus den Ausgangsverbindungen in einem geeigneten Lösungsmittel in Gegenwart von 0₂, NO und/oder in Gegenwart einer der als Katalysator beanspruchten Verbindungen langsam eine Lösung etwa äquimolarer Mengen der starken Base zufügt und nach vollständigem Umsatz das Reaktionsgemisch in an sich für Horner-Emmons-Reaktionen bekannter Weise aufarbeitet. Auch hierbei ist es aber in vielen Fällen möglich, daß man den alkalistabileren Reaktanden mit der Base vorlegt und den anderen Reaktanden langsam zu dem Gemisch zufügt.

Besondere Bedeutung hat das erfindungsgemäße Verfahren für die Herstellung von einem β-Apo-8'-carotinsäureester der allgemeinen Formel VI 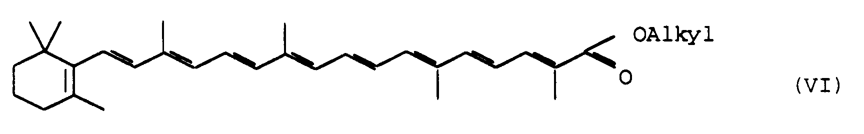 mit einem all-E-Gehalt größer als 85 % durch Umsetzen von β-Apo-12'-carotinal (C₂₅-Aldehyd) mit einem all-E-Gehalt größer als 85 % mit einem 4-Dialkylphosphono-2-methyl-2-butensäurealkylester in einer Horner-Emmons-Reaktion,
für die Herstellung von Neurosporaxanthinsäureestern der allgemeinen Formel VII (C₃₅-Ester) 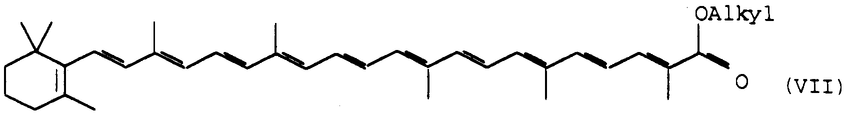 mit einem all-E-Gehalt größer als 85 % durch Umsetzen von β-Apo-8'-carotinal (C₃₀-Aldehyd) mit einem all-E-Gehalt größer als 85 % mit einem 4-Dialkylphosphono-2-methyl-2-butensäurealkylester in einer Horner-Emmons-Reaktion und
die Herstellung von Citranaxanthin mit einem all-E-Gehalt größer als 85 % durch Umsetzen von β-Apo-8'-carotinal mit einem all-E-Gehalt von größer als 85 % mit Aceton in einer Aldolkondensation.

Da bei den genannten langkettigen Polyencarbonylverbindungen der allgemeinen Formel III die all-E-Isomeren in geeigneten Lösungsmitteln wesentlich besser und leichter kristallisieren, werden im allgemeinen auch höhere Ausbeuten an der gewünschten Polyencarbonylverbindung in kristalliner Form erhalten. Bei Einsatz von stabilen Radikalen, Chinonen, Chinonderivaten oder Coenzym-Q10-hydrochinon als Katalysator verbleiben diese bei der Aufarbeitung im allgemeinen praktisch quantitativ in der Waschlauge oder in der Mutterlauge.

Mit Hilfe des erfindungsgemäßen Verfahrens können Polyencarbonylverbindungen der allgemeinen Formel III, wie die als Lebensmittelfarbstoffe sehr begehrten β-Apo-8'-carotinsäureester, Neurosporaxanthinsäureester und Citranaxanthin mit einem all-E-Gehalt von bis zu 95 % erhalten werden, wenn die längerkettigen Ausgangsprodukte weitgehend in all-E-Konfiguration vorliegen.

### Beispiel 1

### Herstellung von β-Apo-8'-carotinsäureethylester (C₃₀-Ethylester) durch Horner-Emmons-Reaktion

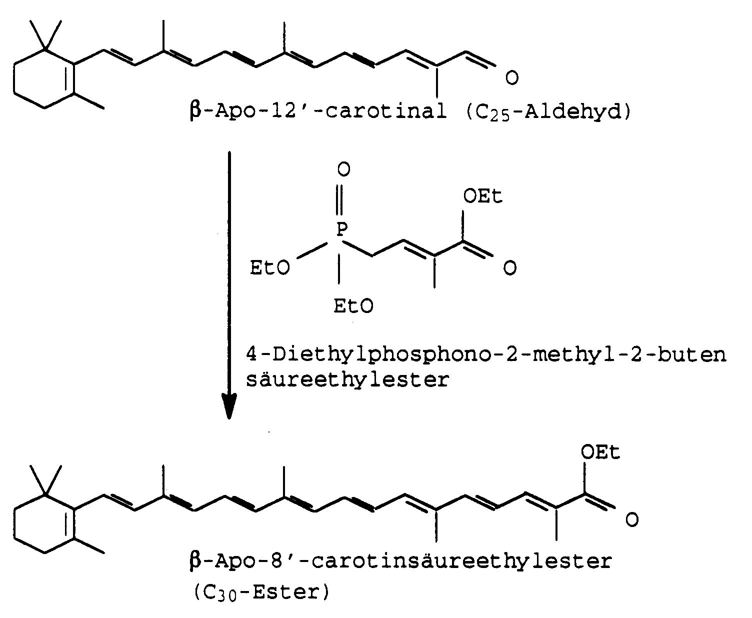

### Variante A (Einsatz von 1 Mol-% 4-OH-TEMPO/Reaktionsdauer: 5 Stunden)

Zu der Maische von 350.5 g (1 mol) kristallinem β-Apo-12'-carotinal (C₂₅-Aldehyd) in 1200 ml technischem Heptan wurden bei 25°C unter Stickstoff-Atmosphäre 1,72 g (10 mmol) 4-Hydroxy-2,2,6,6-tetramethyl-piperidin-N-oxyl (4-OH-TEMPO) in 5 ml Ethanol gegeben. Danach wurden 289 g (1,05 mol) eines 96 %igen 4-Diethylphosphono-2-methyl-2E-butensäureethylesters (C₅-Ester-phosphonat) zugesetzt. Innerhalb von 4 Stunden (h) wurden bei 25-30°C 500 ml einer 20 %igen Natriumethylatlösung (1,28 mol; Dichte 0,873 kg/l) unter guter N₂-Begasung (ca. 8 l/h) zudosiert. Anschließend wurde bei 25°C 1 h nachgerührt. Der all-E-Anteil im C₃₀-Ester betrug nach HPLC 92,2 %.

Anschließend wusch man die organische Phase mit verdünnter wäßriger Schwefelsäure und 60 %igem wäßrigem Methanol.

Nach Zugabe von Methanol wurde der erhaltene β-Apo-8'-carotinsäureethylester abfiltriert und zweimal mit Methanol gewaschen. Die Trocknung erfolgte unter N₂ bei 50°C bis zur Gewichtskonstanz.

Ausbeute: 390,2 g (84,7 %); all-E-Gehalt: 99,7 %.

### Variante B (Einsatz von 0,5 Mol-% TEMPO; Reaktionsdauer 76 h)

Zu der Maische von 350,5 g (1 mol) kristallinem C₂₅-Aldehyd in 1200 ml technischem Heptan wurden bei 25°C unter Stickstoff-Atmosphäre 0,79 g (5 mmol) 2,2,6,6-Tetramethyl-piperidin-N-oxyl (TEMPO) in 5 ml Ethanol gegeben. Danach wurden 289 g (1,05 mol) 96 %iges C₅-Ester Phosphonat zugesetzt.

Innerhalb von 76 h wurden bei 25°C 500 ml einer 20 %igen NaOEt-Lösung (1,28 Mol; Dichte 0,873 kg/l) unter guter N₂-Begasung zudosiert. Der all-E-Anteil im C₃₀-Ester betrug nach HPLC 92,3 %. Die Aufarbeitung erfolgte analog Variante A.

Ausbeute: 380,4 g (82,6 %); all-E-Gehalt: 99,3 %.

Zur Bestimmung des Einflusses der Menge an eingesetztem TEMPO bzw. 4-Hydroxy-TEMPO(4-OH-TEMPO) wurde der C₂₅-Aldehyd β-Apo-12'-carotinal analog Beispiel 1 Varianten A und B in Gegenwart der aus der Tabelle 1 bzw. der Tabelle 2 ersichtlichen Menge an TEMPO bzw. 4-Hydroxy-TEMPO mit dem C₅-Ester-Phosphonat umgesetzt und in dem erhaltenen Reaktionsgemisch der all-E-Anteil im C₃₀-Ester mittels HPLC bestimmt. Die Ergebnisse sind in den Tabellen 1 und 2 zusammengestellt.

**Tabelle 1**

| Einfluß der TEMPO-Menge auf das E/Z-Verhältnis in der beschriebenen Horner-Emmons-Reaktion | | | | | | |
|---|---|---|---|---|---|---|
| Beispiel 1 | Variante (Reaktionszeit) | Fahrweise | C₅-Ester-Phosphonat: Reinheit (E/Z-Verhältnis) | TEMPO [Mol-% bez. C₂₅-aldehyd] | all-E-Gehalt von C₂₅-Aldehyd | all-E-Gehalt des C₃₀-Esters |
| a | A (5 h) | unter N₂ | 96 % (18/1) | 5 Mol-% | 99,0 % | 94,6 % |
| b | A (5 h) | unter N₂ | 96 % (18/1) | 5 Mol-% | 99,0 % | 94,3 % |
| c | A (5 h) | unter N₂ | 97 % (7/1) | 5 Mol-% | 99,0 % | 94,9 % |
| d | A (5 h) | unter N₂ | 96 % (18/1) | 3 Mol-% | 99,0 % | 93,5 % |
| e | A (5 h) | unter N₂ | 96 % (18/1) | 1 Mol-% | 99,0 % | 88,5 % |
| f | A (5 h) | unter N₂ | 96 % (18/1) | 0,5 Mol-% | 99,0 % | 77,5 % |
| g | B (76 h) | unter N₂ | 96 % (18/1) | 0,5 Mol-% | 98,6 % | 92,3 % |

**Tabelle 2**

| Einfluß der 4-OH-TEMPO-Menge auf das E/Z-Verhältnis in der beschriebenen Horner-Emmons-Reaktion | | | | | | |
|---|---|---|---|---|---|---|
| Beispiel 1 | Variante (Reaktionszeit) | Fahrweise | C₅-Ester-Phosphonat: Reinheit (E/Z-Verhältnis) | 4-OH-TEMPO [Mol-% bez. C₂₅-Aldehyd-] | all-E-Gehalt von C₂₅-Aldehyd | all-E-Gehalt von C₃₀-Ester |
| h | A (5 h) | unter N₂ | 96 % (18/1) | 5 Mol-% | 99,0 % | 95,0 % |
| i | A (5 h) | unter N₂ | 96 % (18/1) | 3 Mol-% | 99,0 % | 94,7 % |
| j | A (5 h) | unter N₂ | 96 % (18/1) | 1 Mol-% | 99,0 % | 92,2 % |
| k | A (5 h) | unter N₂ | 96 % (18/1) | 0,5 Mol-% | 99,0 % | 77,4 % |

Analog Beispiel 1, Variante A, wurde der C₂₅-Aldehyd ohne Zusatz von Sauerstoff oder stabilen Radikalen (Vergleichsversuche) bzw. in Gegenwart von verschiedenen stabilen Radikalen oder Radikalfängern mit dem C₅-Esterphosphonat umgesetzt und im erhaltenen Reaktionsgemisch der all-E-Gehalt im C₃₀-Ester mittels HPLC bestimmt. Die Ergebnisse sind in Tabelle 3 zusammengestellt. Versuche mit unwirksamen stabilen Radikalen bzw. Radikalfängern oder Antioxidantien sind Vergleichsversuche und sind mit einem Sternchen * versehen. Der Sauerstoff wurde in allen Versuchen in Form von sogenannter Magerluft (95 % N₂ + 5 % O₂) verwendet. Im Falle der Verwendung von NO-Gas wurden 120 ml NO in 5 bis 10 min eingeleitet, wobei dafür gesorgt wurde, daß das NO den Reaktionsraum während der Basenzugabe nicht verlassen kann.

Der Name 3-Carboxy-proxyl bzw. 3-Carbamoyl-proxyl steht dabei für 3-Carboxy- bzw. 3-Carbamoyloxy-2,2,5,5-tetramethyl-pyrrolidin-N-oxyl und der Name 3-Carbamoyl-doxyl für 3-Carbamoyl-2,5-dihydro-2,2,5,5-tetramethyl-pyrrol-N-oxyl.

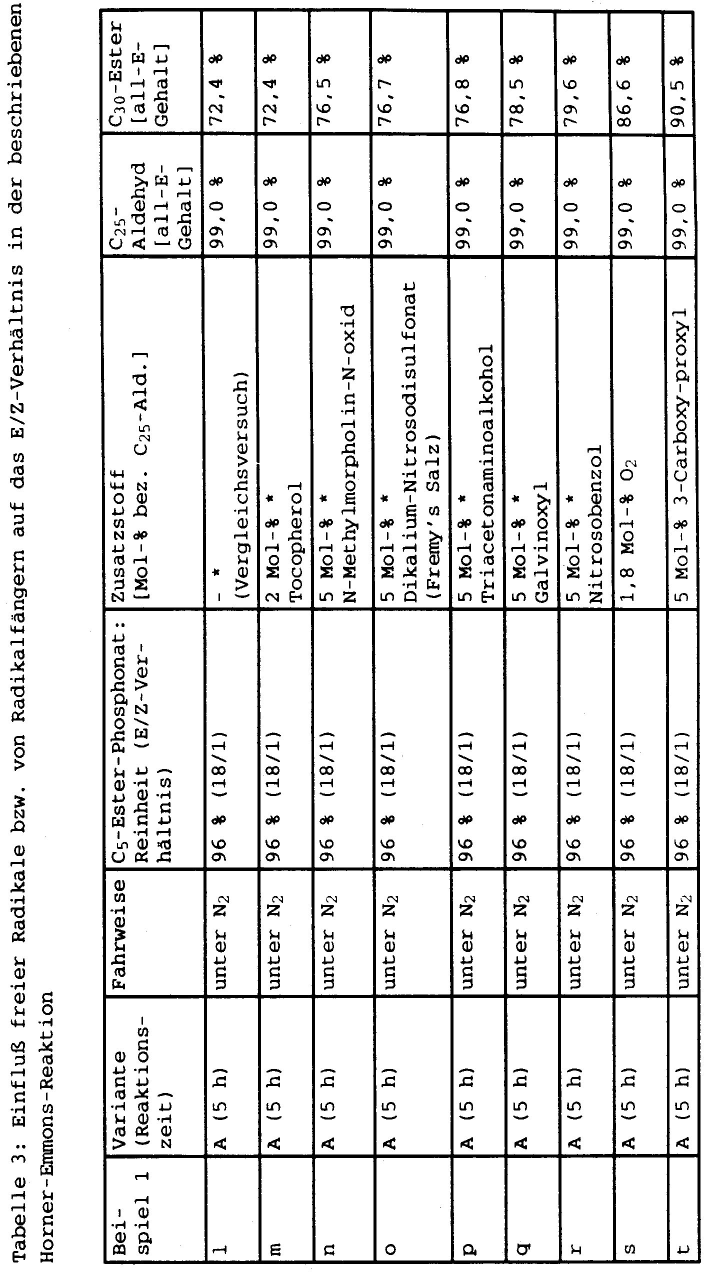

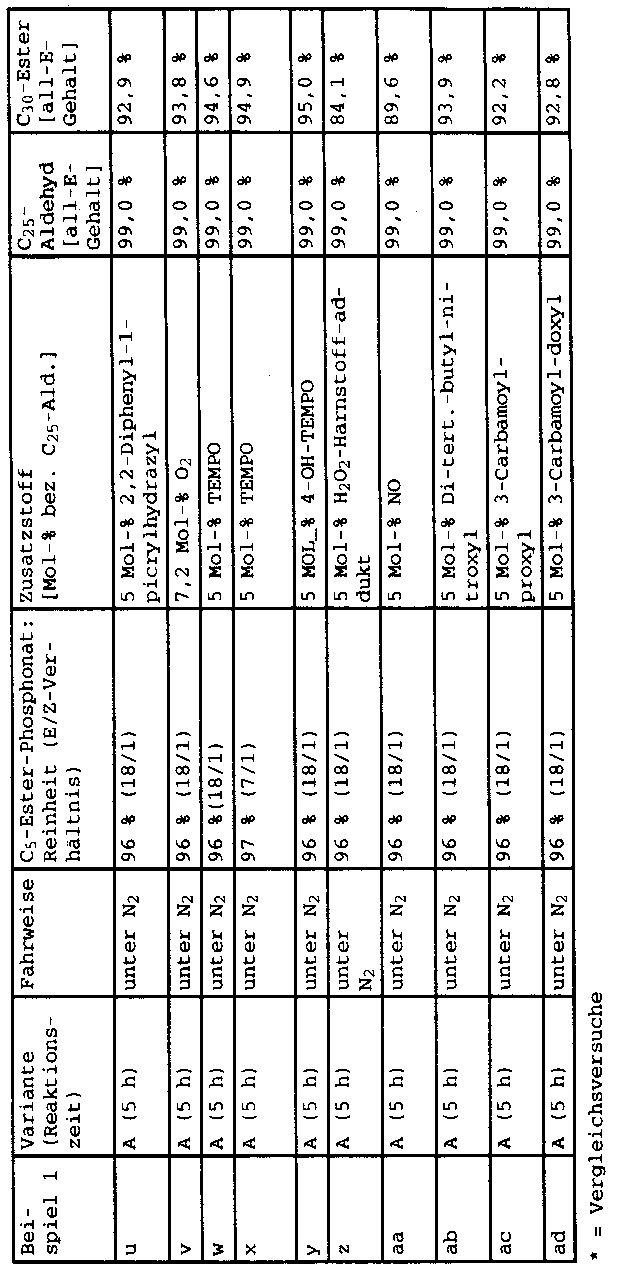

### Beispiel 2

### Herstellung von β-Apo-8'-carotinsäureethylester

a) in Gegenwart von O₂
   70 g β-Apo-12'-carotinal (E-Gehalt 98 %) wurden in 300 ml Heptan suspendiert und mit 60 g eines 96 %igen 4-Diethylphosphono-2-methyl-2E-butensäureethylester versetzt. Unter Rühren und Einleiten von 10 l/h "Magerluft" (Gemisch von N₂ und Luft im Verhältnis 3 : 1) wurden innerhalb von 4 h bei 20 - 25°C 110 ml einer 20 %igen Natriumethylatlösung zugetropft. Anschließend wurde die organische Phase mit verdünnter Schwefelsäure sowie mit 60 %igem wäßrigem Methanol gewaschen. Der all-E-Anteil im erhaltenen β-Apo-8'-carotinsäureethylester betrug nach HPLC 94,7 %, der Z-Anteil 5,3 %.
   Nach Abkühlen auf 0°C, Absaugen und Trocknen der Kristalle im N₂-Strom erhielt man 79,7 g an reinem kristallinem all-E-β-Apo-8'-carotinsäureethylester (all-E-Gehalt: >98 %). Dies entspricht einer Ausbeute von 86,6 % der Theorie, bezogen auf β-Apo-12'-carotinal.
b) Vergleichsbeispiel (unter N₂-Atmosphäre)
   Es wurde gearbeitet wie oben unter a) beschrieben, nur daß anstelle von "Magerluft" 10 l/h Stickstoff in das Reaktionsgemisch eingeleitet wurden. Der all-E-Anteil im erhaltenen β-Apo-8'-carotinsäureethylester betrug nach HPLC 77,4 %, der Gehalt an Z-β-Apo-8'-carotinsäureethylester-Isomeren 22,6 %.
   Unter gleichen Kristallisationsbedingungen konnten nur 63 g eines reinen kristallinen all-E-β-Apo-8'-carotinsäureethylesters erhalten werden. Das entspricht einer Ausbeute von nur 68,5 %, bezogen auf eingesetztes β-Apo-12'-carotinal.

### Beispiel 3

### Herstellung von β-Apo-4'-Carotinsäureethylester(C₃₅-Ester) durch Horner-Emmons-Reaktion

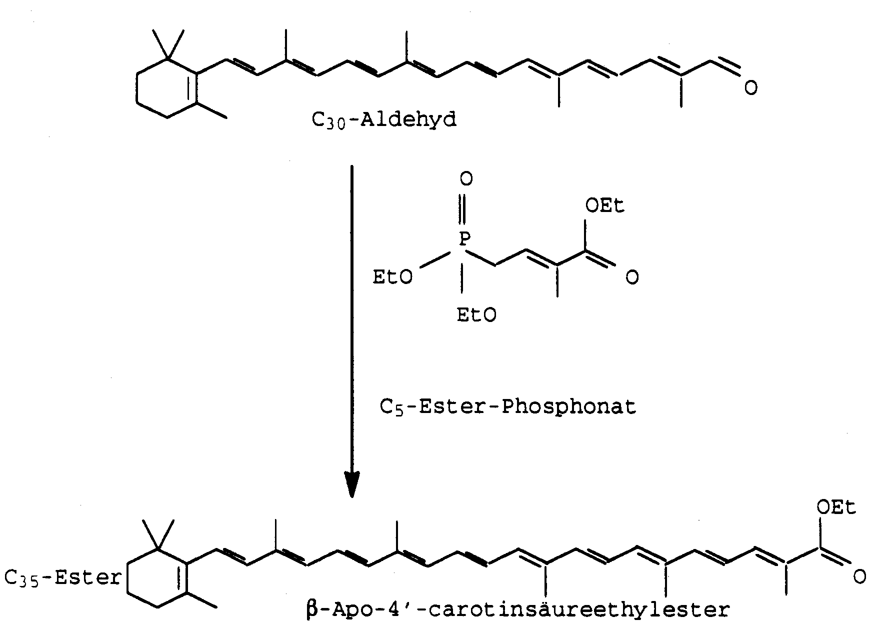

Zu der Maische von 419,6 g (1 mol) eines 99,3 % reinen kristallinen C₃₀-Aldehyds in 1200 ml technischem Heptan wurden bei 25°C unter Stickstoff-Atmosphäre 7,8 g (50 mmol) TEMPO gegeben. Danach wurden 289 g (1,05 mol) eines 96 %igen C₅-Ester-Phosphonats zugesetzt.
Innerhalb von 4 h wurden bei 25-30°C 500 ml einer 20 %igen NaOEt-Lösung (1,28 mol; Dichte 0,873 kg/l) unter guter N₂-Begasung (ca. 8 l/h) zudosiert. Während der Reaktion entstand eine sehr kräftige Kristallmaische.
Der all-E-Anteil im C₃₅-Ester betrug nach Ende der Basenzugabe laut HPLC 94,0 %.

Anschließend wusch man die abgetrennte organische Phase mit verdünnter wäßriger Schwefelsäure und mit einem Gemisch aus Methanol und verdünnter wäßriger Schwefelsäure.

Nach Zugabe von Heptan und Methanol wurde abfiltriert und mit Wasser gewaschen. Trocknung: unter N₂ bei 50°C/1 mbar bis zur Gewichtskonstanz.

Ausbeute: 495,2 g (94,0 %) Kristallisat; all-E-Gehalt: 97,5 %; Fp. 140-141°C
E¹ (1 %, 1 cm): 2656 (Cyclohexan; 479 nm); Gehalt: 98,4 % ber. mit E¹ = 2700
Ein etwa gleiches Ergebnis erhielt man bei Verwendung von 5 Litern (L) "Magerluft" pro Stunde und Mol anstelle von TEMPO.

### Beispiel 4

### Herstellung von Citranaxanthin durch Aldolkondensation

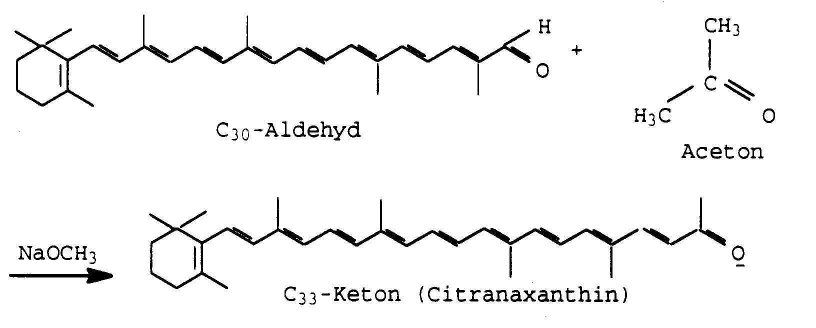

Jeweils 41,67 g (0,1 mol) C₃₀-Aldehyd wurden entsprechend den Angaben in der folgenden Tabelle unter "Magerluft"- oder Stickstoffbegasung (10 Liter/h) bei Raumtemperatur (RT) in einem Gemisch aus 417 ml Aceton und 208 ml Heptan suspendiert. Die Suspension wurde auf 40°C erwärmt und unter "Magerluft"- oder N₂-Begasung innerhalb von 6 h 63 ml einer 1 %igen Lösung der aus der Tabelle ersichtlichen Base in Methanol zugetropft. Stündlich wurden Proben zur Bestimmung des Reaktionsverlaufs mittels HPLC abgenommen.

Das auskristallisierte Citranaxanthin wurde abgesaugt und mit Methanol gewaschen. Die Trocknung erfolgte in N₂-Strom bei 50°C/1 mbar. Die Ausbeuten wurden nur bei den Reaktionsansätzen bestimmt, die ohne nennenswerte Isomerisierung verliefen.

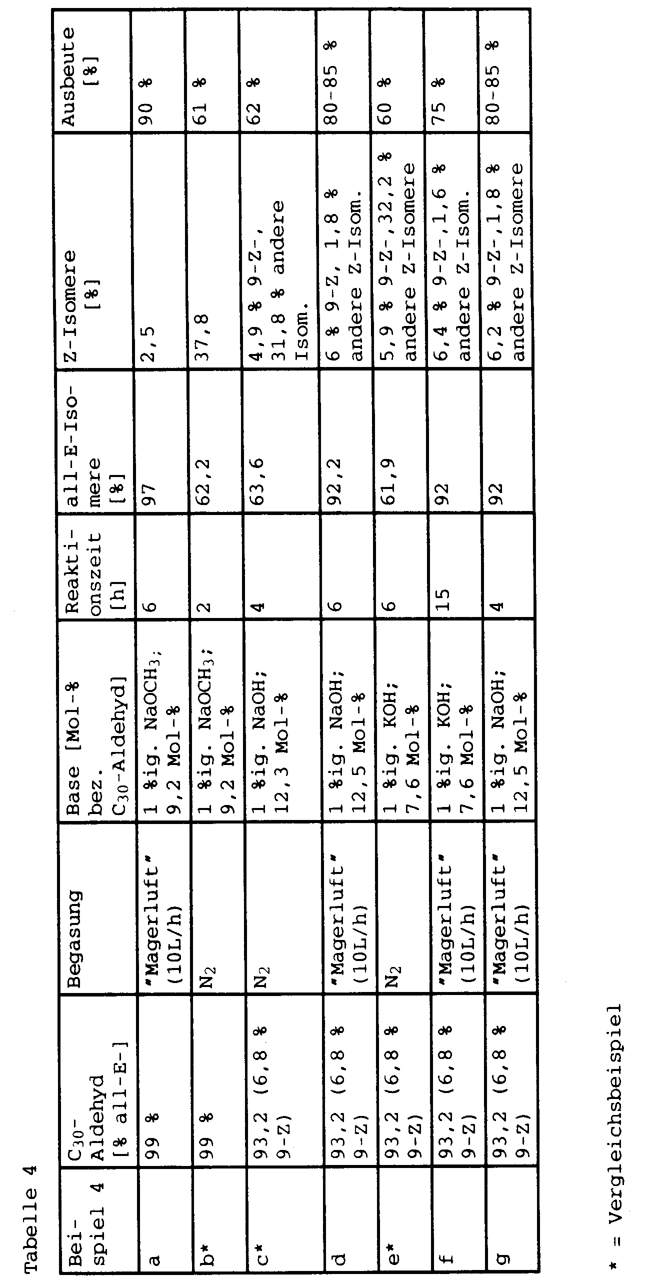

### Beispiel 5

### Herstellung von Citranaxanthin

Jeweils 33 g (0,08 mol) C₃₀-Aldehyd (all-E-Gehalt 98 %) wurden in 575 ml Aceton suspendiert, ggf. 2 g BHT (butyliertes Hydroxytoluol als Antioxidans) und die aus der folgenden Tabelle 5 ersichtliche Menge an "TEMPO" zugesetzt und das erhaltene Reaktionsgemisch auf 43°C erwärmt. Bei dieser Temperatur wurde innerhalb der auf Tabelle 5 angegebenen Reaktionszeit 5,2 g einer 50 gew.-%igen wäßrigen NaOH (0,07 mmol) zugetropft.

Anschließend wurde das Reaktionsgemisch in 30 min auf 20°C abgekühlt, noch 30 min bei 20°C nachgerührt. Der Gehalt des Reaktionsgemisches an all-E-Citranaxanthin sowie der Gehalt an anderen Citranaxanthin-Isomeren wurde mittels HPLC bestimmt und ist in Tabelle 5 angegeben.

Anschließend wurde über eine Nutsche filtriert. Der Filterkuchen wurde mit Methanol gewaschen und bei ca. 50°C im Trockenschrank getrocknet.

**Tabelle 5**

| Beispiel 4 | "TEMPO" [Mol-%] | Begasung | Base | Reaktionszeit [h (°C)] | all-E-Isomeres [%] | andere Isomere [%] |
|---|---|---|---|---|---|---|
| a* | - (+2g BHT) | N₂ | NaOH | 3 (43°C) | 51,4 | 47,9 |
| b | 5 (+2g BHT) | N₂ | NaOH | 3 (43°C) | 71,7 | 26,9 |
| c* | - | N₂ | NaOH | 1 (43°C) | 50,7 | 48,5 |
| d | 5 | N₂ | NaOH | 3 | 62,9 | 35,5 |
| e | 30 | N₂ | NaOH | 3 | 67,0 | 31,3 |
| f | 100 | N₂ | NaOH | 3 | 73,5 | 24,7 |

| | | | | | | |
|---|---|---|---|---|---|---|
| * = Vergleichsbeispiel | | | | | | |

### Beispiel 6

### Herstellung von β-Apo-8'-carotinsäureethylester durch Horner-Emmons-Reaktion in Gegenwart von Bis(1-Oxyl-2,2,6,6-tetramethyl-piperidin-4-yl)-sebacat

Zu der Maische von 25.05 g (100 mmol) kristallinem β-Apo-12'-carotinal in 120 ml technischem Heptan wurden bei 25°C unter Stickstoffatmosphäre 1,28 g (2,5 mmol) Bis(1-Oxyl-2,2,6,6,-tetramethyl-piperidin-4-yl)-sebacat in 5 ml Ethanol gegeben. Danach wurden 30,3 g (110 mmol) eines 96 %igen C₅-Ester-phosphonats zugesetzt. Innerhalb von 4 h wurden bei 25-30°C 50 ml 20 %ige Natriumethylatlösung (128 mmol) unter N₂-Begasung zudosiert. Anschließend wurde bei 25°C 1 h nachgerührt. Der all-E-Anteil im C₃₀-Ester betrug nach HPLC 94,9 %.

Anschließend wusch man die organische Phase mit verdünnter wäßriger Schwefelsäure und 60 %igem Methanol.

Nach Zugabe von Methanol wurde der erhaltene β-Apo-8'-carotinsäureethylester abfiltriert und zweimal mit Methanol gewaschen. Die Trocknung erfolgte bis zur Gewichtskonstanz.

Ausbeute: 39,5 g (85,8 %); all-E-Gehalt: 99,8 %.

### Beispiel 7

### Herstellung von β-Apo-8'-carotinsäureethylester (C₃₀-Ethylester) durch Horner-Emmons-Reaktion in Gegenwart von 2,2,4-Trimethyl-6(H)-chinolon

Zu der Maische von 350.5 g (1 mol) kristallinem β-Apo-12'-carotinal (C₂₅-Aldehyd) in 1200 ml technischem Heptan wurden bei 25°C unter Argon-Atmosphäre 1,96 g (10 mmol) 2,2,4-Trimethyl-6(H)-chinolon in 5 ml Ethanol gegeben. Danach wurden 302,8 g (1,1 mol) eines 96%igen 4-Diethylphosphono-2-methyl-2E-butensäureethylesters (C₅-Ester-phosphonat) zugesetzt. Innerhalb von 4 Stunden (h) wurden bei 25-30°C 500 ml einer 20 %igen Natriumethylatlösung (1,28 mol) unter Argon-Begasung zudosiert. Anschließend wurde auf 55°C erhitzt. Der all-E-Anteil im C₃₀-Ester betrug nach HPLC 94,8%.

Anschließend wusch man die organische Phase bei 55°C mit verdünnter wäßriger Schwefelsäure und 60 %igem wäßrigem Methanol.

Nach Zugabe von 1 l Methanol wurde auf -5°C abgekühlt und der erhaltene β-Apo-8'-carotinsäureethylester abfiltriert und mit Methanol gewaschen. Die Trocknung erfolgte unter N₂ bei 50°C bis zur Gewichtskonstanz.

Ausbeute: 401,3 g (87,1 %); all-E-Gehalt: 98,9 %.

### Beispiel 8

### Herstellung von β-Apo-8'-carotinsäureethylester (C₃₀-Ethylester) durch Horner-Emmons-Reaktion in Gegenwart von Chinonen, Chinonderivaten und Coenzym-Q10-hydrochinon

Zu der Maische von 35,05 g (0,1 mol) C₂₅-Aldehyd in 120 ml technischem Heptan wurden bei 25°C unter Argon-Atmosphäre die aus Tabelle 6 ersichtlichen Mengen an dem aus der Tabelle 6 ersichtlichen Zusatzstoff in 2 ml Ethanol gegeben. Danach wurden 30,3 g (110 mmol) eines 96%igen C₅-Ester-phosphonats mit dem aus der Tabelle 1 ersichtlichen E/Z-Isomerenverhältnis zugesetzt. Innerhalb von 4 h wurden bei 25-30°C 50 ml einer 20%igen Natriumethylatlösung (128 mmol) unter Argon-Begasung zudosiert. Anschließend wurde bei 25°C 1 h nachgerührt. Der all-E-Anteil im C₃₀-Ester wurde jeweils mittels HPLC bestimmt. Die Ergebnisse sind in Tabelle 6 dargestellt.

**Tabelle 6**

| Einfluß von Chinonen, Chinoniminen und Coenzym Q10-Hydrochinon auf das E/Z-Verhältnis in der beschriebenen Horner-Emmons-Reaktion | | | | | |
|---|---|---|---|---|---|
| Beispiel | Fahrweise | C₅-Ester-Phosphonat: Reinheit (E/Z-Verhältnis) | Zusatzstoff [Mol-% bez. C₂₅-Aldehyd] | C₂₅-Aldehyd [all-E-Gehalt] | C₃₀-Ester [all-E-Gehalt] |
| 2a | unter Argon | 96 % (25/1) | -* (Vergleichsversuch) | 99,0 % | 76 % |
| 2b | unter Argon | 96 % (25/1) | 5 Mol-% Trimethyl-1,4-benzochinon (TMC) | 99,0 % | 90,2 % |
| 2c | unter Argon | 96 % (25/1) | 5 Mol-% Coenzym Q10 | 99,0 % | 91,7 % |
| 2d | unter Argon | 96 % (25/1) | 5 Mol-% Tetrachlor-1,4-benzochinon | 99,0 % | 91,6 % |
| 2e | unter Argon | 96 % (25/1) | 5 Mol-% Tocochinon-acetat | 99,0 % | 90,3 % |
| 2f | unter Argon | 96 % (25/1) | 5 Mol-% Coenzym Q0 | 99,0 % | 88 % |
| 2g | unter Argon | 96 % (25/1) | 5 Mol-% 2-Methyl-1,4-naphthochinon | 99,0 % | 88 % |
| 2h | unter Argon | 96 % (25/1) | 1 Mol-% 2,2,4-Trimethyl-6(H)-chinolon | 99,0 % | 94,8 % |
| 2i | unter Argon | 96 % (18/1) | 6 Mol-% 2,2,4-Trimethyl-6(H)-chinolon | 98,7 % | 93,7 % |
| 2k | unter Argon | 96 % (25/1) | 5 Mol-% Coenzym-Q10-Hydrochinon | 99,0 % | 88,4 % |

### Beispiel 9

### Herstellung von β-Apo-4'-carotinsäureethylester (C₃₅-Ester) durch Horner-Emmons-Reaktion in Gegenwart von Chinonen, Chinonderivaten und Coenzym-Q10-hydrochinon

41,67 g (100 mmol) C₃₀-Aldehyd wurden in 400 ml technischem Heptan suspendiert und bei Raumtemperatur (RT) unter Argon-Atmosphäre mit 30,3 g (110 mmol) 96 %igem C₅-Ester-phosphonat und 5 mmol der in Tabelle 7 angegebenen Zusatzstoffe versetzt. In 4 h wurden 50 ml einer 20%igen Natriumethylat-Lösung zudosiert. Es wurde 16 h bei RT nachgerührt. Die all-E-Gehalte im Produkt wurden per HPLC bestimmt und in Tabelle 7 zusammengestellt.

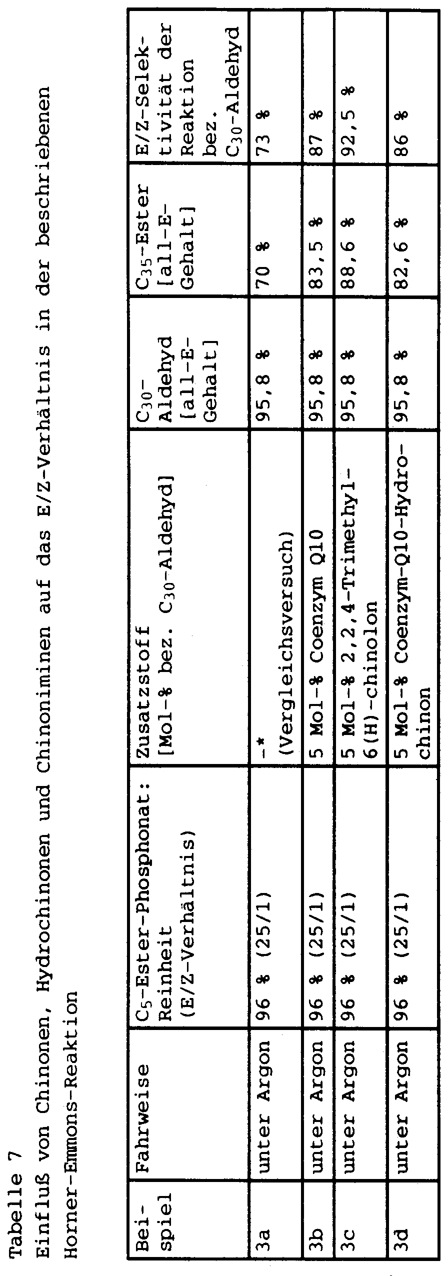

## Patentansprüche

1. Verfahren zur Herstellung von Polyencarbonylverbindungen mit hohem all-E-Gehalt sowie von deren Acetalen bzw. Ketalen durch eine Horner-Emmons-Reaktion oder eine Aldolkondensation einer geeigneten Carbonylverbindung mit einem geeigneten Dialkylphosphonat bzw. einer geeigneten CH-aciden Verbindung, dadurch gekennzeichnet, daß man die Umsetzung zum Zwecke der bevorzugten Bildung einer Doppelbindung mit E-Konfiguration und um die E-Konfiguration der Doppelbindungen in den Ausgangsverbindungen möglichst vollständig zu erhalten in Gegenwart von Sauerstoff oder einem Sauerstoff-Inertgasgemisch, oder in Gegenwart von Stickstoffmonoxid oder einem Stickstoffmonoxid-Inertgasgemisch und/oder in Gegenwart von einem stabilen Radikal der allgemeinen Formel I 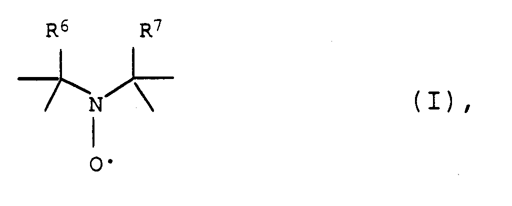 in der R⁶ und R⁷ für eine C₁- bis C₄-Alkylgruppe stehen oder aber R⁶ und R⁷ zusammen für eine Ethylengruppe, Propylengruppe, Vinylengruppe oder Propenylengruppe stehen, die durch Alkyl-, Aryl-, Hydroxyl-, Alkyloxy-, Silyloxy-, Oxo-, Amino-, Mercapto-, Alkylmercapto-, Cyano-, Carboxyl-, Aminocarbonyl-(Carbamoyl-), Heteroaryl oder Alkylcarbonyloxy-Gruppen substituiert sein können
und/oder in Gegenwart eines stabilen Radikals der allgemeinen Formel II 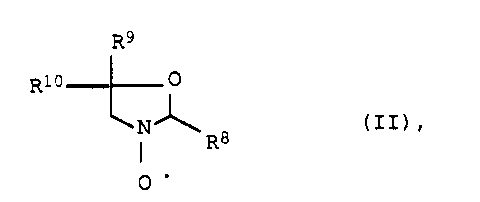 in der R⁸, R⁹ und R¹⁰ die oben für R⁶ und R⁷ angegebener Bedeutung haben können
und/oder in Gegenwart von dem stabilen Radikal 2,2-Diphenyl-1-picrylhydrazyl oder einem Wasserstoffperoxid-Harnstoffaddukt, und/oder in Gegenwart von Chinonen, Chinonderivaten oder Coenzym-Q10-Hydrochinon durchführt,
wobei man den Sauerstoff, das Stickstoffmonoxid, die stabilen Radikale bzw. das Wasserstoffperoxid-Harnstoffaddukt und die Chinone, Chinonderivate oder Coenzym-Q10-hydrochinon in Mengen von 0,3 bis 10 Mol-%, bezogen auf die eingesetzte carbonylverbindung verwendet.

2. Verfahren gemäß Anspruch 1, dadurch gekennzeichnet, daß man als stabiles Radikal der allgemeinen Formel I Di-tert.-butylamin-N-oxyl der Formel Ia 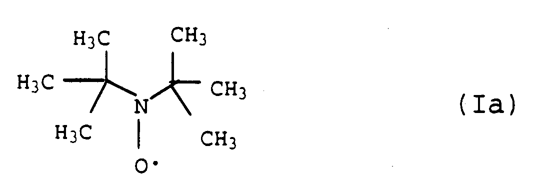 verwendet.

3. Verfahren gemäß Anspruch 1, dadurch gekennzeichnet, daß man als stabiles Radikal der allgemeinen Formel I ein 2,2,6,6-Tetramethyl-piperidin-N-oxyl der Formel Ib 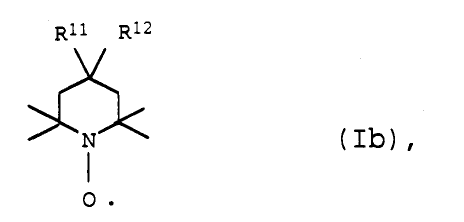 in der R¹¹ für Wasserstoff
und R¹² für Wasserstoff, eine Hydroxyl-, Alkyloxy-, Acetoxy-, Amino-, Cyano- oder eine Carboxylgruppe, oder aber R¹¹ und R¹² zusammen für eine Oxogruppe stehen, verwendet.

4. Verfahren gemäß Anspruch 1, dadurch gekennzeichnet, daß man als stabiles Radikal der allgemeinen Formel I ein 2,2,5,5-Tetramethyl-pyrrolidin-N-oxyl der Formel Ic 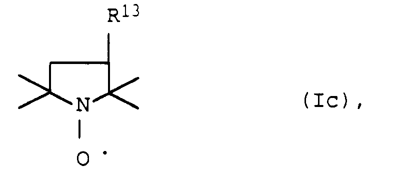 in der R¹³ für Wasserstoff oder eine der in Anspruch 4 für R¹² angegebene Gruppen steht, verwendet.

5. Verfahren gemäß Anspruch 1, dadurch gekennzeichnet, daß man als stabiles Radikal der allgemeinen Formel I ein 2,5-Dihydro-2,2,5,5-tetramethyl-1H-pyrrol-N-oxyl der Formel Id 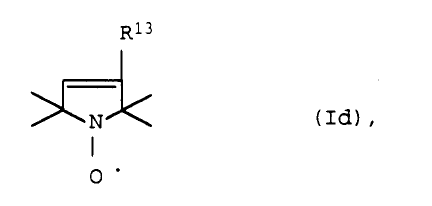 in der R¹³ die in Anspruch 5 angegebene Bedeutung hat, verwendet.

6. Verfahren gemäß Anspruch 1, dadurch gekennzeichnet, daß man die Umsetzung in Gegenwart von Chinonen oder Chinonderivaten der allgemeinen Formeln X oder XI 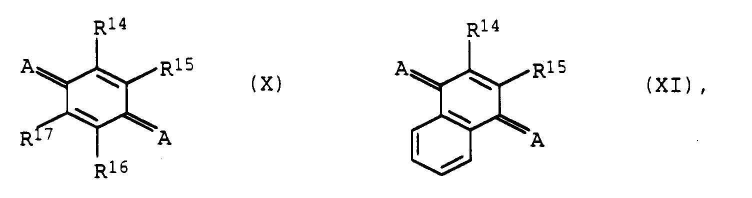 in denen A für =0, =NH oder =N-R⁵ steht, worin R⁵ für eine Alkylgruppe steht oder aber R⁵ mit einem der benachbarten Reste R¹ bis R⁴ zusammen einen Alkylenrest mit 2 oder 3 C-Atomen bildet, der durch Halogen, C₁- bis C₄-Alkylgruppen oder C₁-C₄-Alkoxygruppen substituiert sein kann,
und R¹⁴ bis R¹⁷ für Wasserstoff, Halogen oder eine Alkyl-, Alkenyl-, Cycloalkyl-, Alkoxy-, Alkoxycarbonyl-, Cyano-, Acyloxy-, Aryl- oder Heteroarylgruppen stehen, durchführt.

7. Verfahren gemäß Anspruch 1, dadurch gekennzeichnet, daß man die Umsetzung in einem acyclischen, einem cyclischen oder einem aromatischen Kohlenwasserstoff oder Halogenkohlenwasserstoff, in einem Alkanol oder einem polaren aprotischen Lösungsmittel oder einer Mischung von 2 oder mehreren dieser Lösungsmittel durchführt.

8. Verfahren gemäß Anspruch 1, dadurch gekennzeichnet, daß man zur Herstellung von einem β-Apo-8'-carotinsäureester der allgemeinen Formel VI 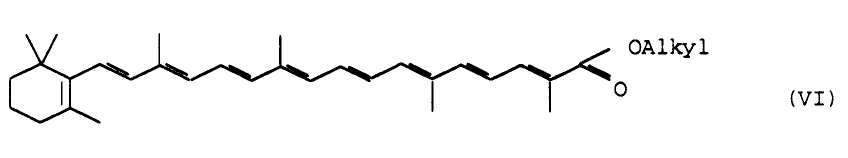 mit einem all-E-Gehalt größer als 85 % β-Apo-12'-carotinal (C₂₅-Aldehyd) mit einem all-E-Gehalt größer als 85 % in Gegenwart von Sauerstoff oder einem Sauerstoff-Inertgas-Gemisch oder Stickstoffmonoxid oder einem Stickstoffmonoxid-Inertgas-Gemisch und/oder in Gegenwart von 2,2,6,6-Tetramethyl-piperidin-N-oxyl (TEMPO), 4-Hydroxy-2,2,6,6-tetramethyl-piperidin-N-oxyl (4-OH-TEMPO), 4-Oxo-2,2,6,6-tetramethyl-piperidin-N-oxyl, 3-Carboxy-2,2,5,5-tetramethyl-pyrrolidin-N-oxyl, 3-Aminocarbonyl-2,2,5,5-tetramethyl-pyrrolidin-N-oxyl, 3-Aminocarbonyl-2,5-dihydro-2,2,5,5-tetramethyl-1H-pyrrol-N-oxyl, Di-tert-butylamin-N-oxyl oder 2,2-Diphenyl-1-picrylhydrazyl als stabilem Radikal Trimethyl-1,4-benzochinon, Coenzym Q10, Tetrachlor-1,4-benzochinon, Tocochinon-acetat, Coenzym Q0 oder 2,2,4-Trimethyl-6(H)-chinolon als Chinon bzw. Chinonderivat mit einem 4-Dialkylphosphono-2-methyl-2-butensäurealkylester in einer Horner-Emmons-Reaktion umsetzt.

9. Verfahren gemäß Anspruch 1, dadurch gekennzeichnet, daß man zur Herstellung von Neurosporaxanthinsäureestern der allgemeinen Formel VII 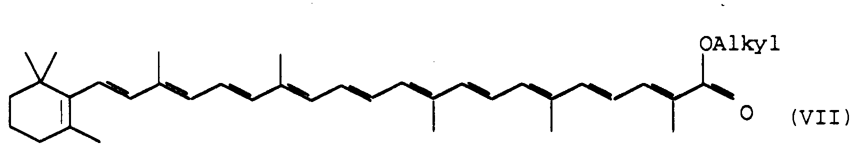 mit einem all-E-Gehalt größer als 85 % β-Apo-8'-carotinal mit einem all-E-Gehalt größer als 85 % in Gegenwart von Sauerstoff oder einem Sauerstoff-Inertgas-Gemisch oder Stickstoffmonoxid oder einem Stickstoffmonoxid-Inertgas-Gemisch und/oder in Gegenwart von einem der in Anspruch 8 genannten stabilen Radikale oder aber mit einem der in Anspruch 8 genannten Chinone oder Chinonderivate mit einem 4-Dialkylphosphono-2-methyl-2-butensäurealkylester in einer Horner-Emmons-Reaktion umsetzt.

10. Verfahren gemäß Anspruch 1, dadurch gekennzeichnet, daß man zur Herstellung von Citranaxanthin der Formel VIII 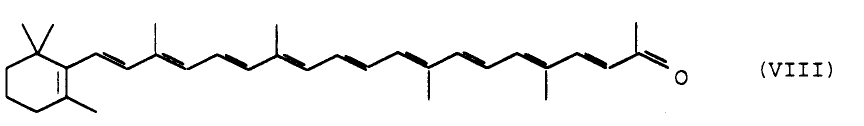 mit einem all-E-Gehalt größer als 85 % β-Apo-8'-carotinal mit einem all-E-Gehalt größer als 85 % in Gegenwart von Sauerstoff oder einem Sauerstoff-Inertgas-Gemisch oder Stickstoffmonoxid oder einem Stickstoffmonoxid-Inertgas-Gemisch mit Aceton in einer Aldolkondensation umsetzt.

## Claims

1. A process for preparing polyenecarbonyl compounds having a high all-E content and their acetals or ketals by a Horner-Emmons reaction or an aldol condensation of a suitable carbonyl compound with a suitable dialkyl phosphonate or of a suitable CH-acidic compound, which comprises carrying out the reaction, for the purposes of the preferred formation of a double bond having E configuration and in order to obtain the E configuration of the double bonds in the starting compounds as completely as possible, in the presence of oxygen or an oxygen-inert gas mixture, or in the presence of nitric oxide or a nitric oxide-inert gas mixture and/or in the presence of a stable radical of the general formula I 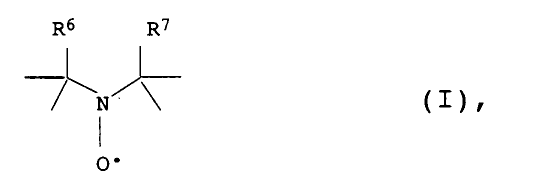 where R⁶ and R⁷ are a C₁- to C₄-alkyl group
or else R⁶ and R⁷ together are an ethylene group, propylene group, vinylene group or propenylene group, which can be substituted by alkyl, aryl, hydroxyl, alkoxy, silyloxy, oxo, amino, mercapto, alkylmercapto, cyano, carboxyl, aminocarbonyl (carbamoyl), heteroaryl or alkylcarbonyloxy groups; and/or in the presence of a stable radical of the general formula II 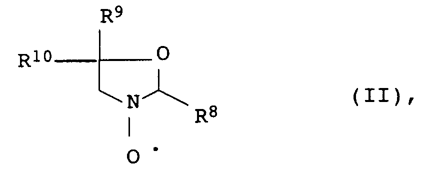 where R⁸, R⁹ and R¹⁰ can have the meanings indicated above for R⁶ and R⁷;
and/or in the presence of the stable radical 2,2-diphenyl-1-picrylhydrazyl or a hydrogen peroxide-urea adduct, and/or in the presence of quinones, quinone derivatives or coenzyme C10 hydroquinone,
the oxygen, the nitric oxide, the stable radicals or the hydrogen peroxide-urea adduct and the quinones, quinone derivatives or coenzyme Q10 hydroquinone being used in amounts from 0.3 to 10 mol %, based on the carbonyl compound employed.

2. A process as claimed in claim 1, wherein the stable radical of the general formula I used is di-tert-butylamine-N-oxyl of the formula Ia

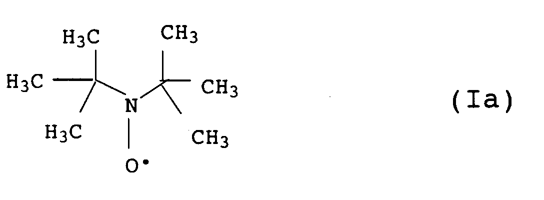

3. A process as claimed in claim 1, wherein the stable radical of the general formula I used is a 2,2,6,6-tetramethylpiperidine-N-oxyl of the formula Ib 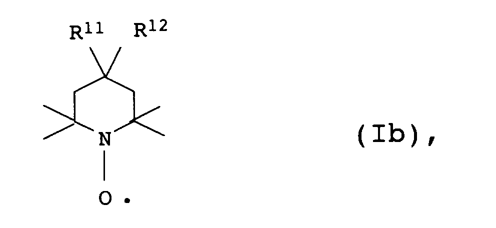 where R¹¹ is hydrogen
and R¹² is hydrogen, or a hydroxyl, alkoxy, acetoxy, amino, cyano or carboxyl group, or else R¹¹ and R¹² together are an oxo group.

4. A process as claimed in claim 1, wherein the stable radical of the general formula I used is a 2,2,5,5-tetramethylpyrrolidine-N-oxyl of the formula Ic 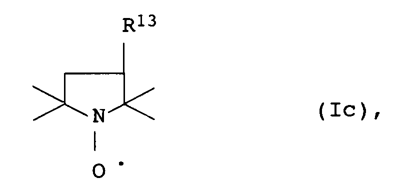 where R¹³ is hydrogen or one of the groups indicated for R¹² in claim 3.

5. A process as claimed in claim 1, wherein the stable radical of the general formula I used is a 2,5-dihydro-2,2,5,5-tetramethyl-1H-pyrrole-N-oxyl of the formula Id 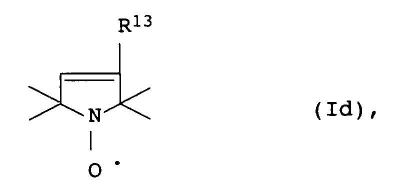 where R¹³ has the meaning indicated in claim 4.

6. A process as claimed in claim 1, wherein the reaction is carried out in the presence of quinones or quinone derivatives of the general formula X or XI 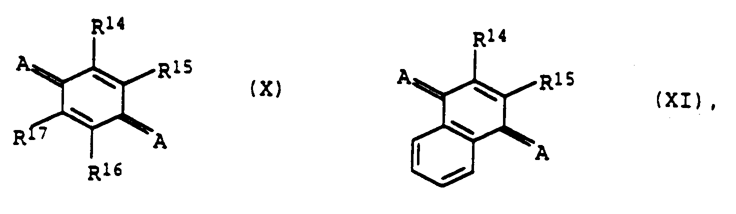 where A is =O, =NH or =N-R⁵, in which R⁵ is an alkyl group or else R⁵ with one of the adjacent radicals R¹ to R⁴ together form an alkylene radical having 2 or 3 C atoms, which can be substituted by halogen, C₁- to C₄-alkyl groups or C₁- to C₄-alkoxy groups,
and R¹⁴ to R¹⁷ are hydrogen, halogen or an alkyl, alkenyl, cycloalkyl, alkoxy, alkoxycarbonyl, cyano, acyloxy, aryl or heteroaryl group.

7. A process as claimed in claim 1, wherein the reaction is carried out in an acyclic, a cyclic or an aromatic hydrocarbon or halohydrocarbon, or in an alcohol or a polar aprotic solvent or a mixture of 2 or more of these solvents.

8. A process as claimed in claim 1, wherein to prepare a β-apo-8'-carotenic acid ester of the general formula VI 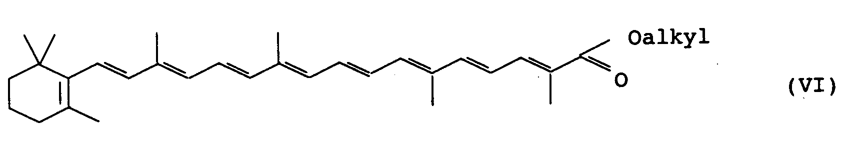 having an all-E content of greater than 85%, β-apo-12'-carotenal (C₂₅ aldehyde) having an all-E content of greater than 85% is reacted with an alkyl 4-dialkylphosphono-2-methyl-2-butenoate in a Horner-Emmons reaction in the presence of oxygen or an oxygen-inert gas mixture or nitric oxide or a nitric oxide-inert gas mixture and/or in the presence of 2,2,6,6-tetramethylpiperidine-N-oxyl (TEMPO), 4-hydroxy-2,2,6,6-tetramethylpiperidine-N-oxyl (4-OH-TEMPO), 4-oxo-2,2,6,6-tetramethylpiperidine-N-oxyl, 3-carboxy-2,2,5,5-tetramethylpyrrolidine-N-oxyl, 3-aminocarbonyl-2,2,5,5-tetramethylpyrrolidine-N-oxyl, 3-aminocarbonyl-2,5-dihydro-2,2,5,5-tetramethyl-1H-pyrrole-N-oxyl, di-tert-butylamine-N-oxyl or 2,2-diphenyl-1-picrylhydrazyl as a stable radical or else trimethyl-1,4-benzoquinone, coenzyme Q10, tetrachloro-1,4-benzoquinone, tocoquinone acetate, coenzyme Q0 or 2,2,4-trimethyl-6(H)-quinolone as a quinone or quinone derivative.

9. A process as claimed in claim 1, wherein to prepare neurosporaxanthinic acid esters of the general formula VII 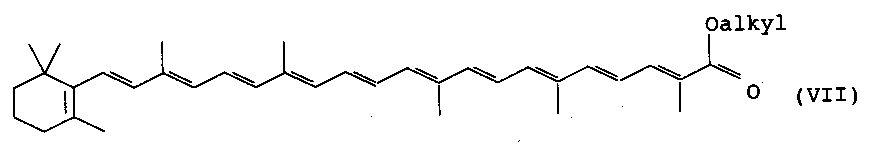 having an all-E content of greater than 85%, β-apo-8'-carotenal having an all-E content of greater than 85% is reacted with an alkyl 4-dialkylphosphono-2-methyl-2-butenoate in a Horner-Emmons reaction in the presence of oxygen or an oxygen-inert gas mixture or nitric oxide or a nitric oxide-inert gas mixture and/or in the presence of one of the stable radicals mentioned in claim 8 or else with one of the quinones or quinone derivatives mentioned in claim 8.

10. A process as claimed in claim 1, wherein to prepare citranaxanthin of the formula VIII 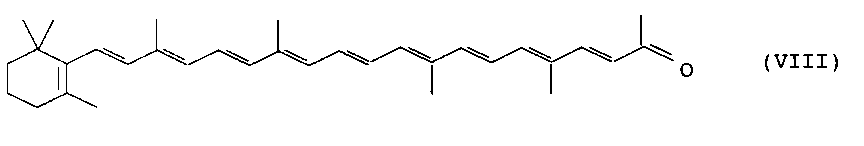 having an all-E content of greater than 85%, β-apo-8'-carotenal having an all-E content of greater than 85% is reacted with acetone in an aldol condensation in the presence of oxygen or an oxygen-inert gas mixture or nitric oxide or a nitric oxide-inert gas mixture.

## Revendications

1. Procédé de préparation de dérivés polyènecarbonylés à forte teneur en isomères tout-E et de leurs acétals ou cétals par une réaction de Horner-Emmons ou une condensation aldolique entre un dérivés carbonylé approprié et un phosphonate de dialkyle approprié ou respectivement un composé approprié à CH acide, caractérisé par le fait que, dans le but de former préférentiellement une double liaison à configuration E et de conserver aussi complètement que possible la configuration E des doubles liaisons des composés de départ, on effectue la réaction en présence d'oxygène ou d'un mélange oxygène-gaz inerte ou en présence de bioxyde d'azote ou d'un mélange bioxyde d'azote-gaz inerte et/ou en présence d'un radical stable de formule générale I 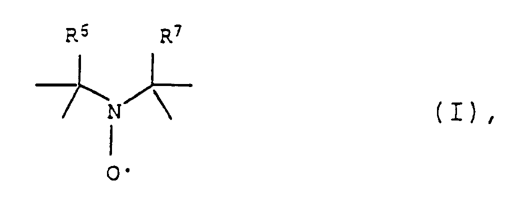 dans laquelle R⁶ et R⁷ représentent chacun un groupe alkyle en C1-C4 ou forment ensemble un groupe éthylène, propylène, vinylidène ou propénylène qui peut être substitué par des groupes alkyle, aryle, hydroxy, alkyloxy, silyloxy, oxo, amino, mercapto, alkylmercapto, cyano, carboxyle, aminocarbonyle (carbamoyle), hétéroaryle ou alkylcarbonyloxy, et/ou en présence d'un radical stable de formule générale II 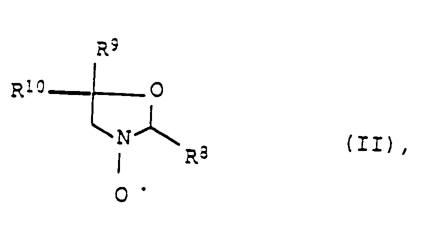 dans laquelle R⁸, R⁹ et R¹⁰ ont les signficiations indiquées ci-dessus pour R⁶ et R⁷, et/ou en présence du radical stable 2,2-diphényl-1-picrylhydrazyle ou d'un adduct peroxyde d'hydrogène-urée et/ou en présence de quinones, de dérivés de quinones ou de la coenzyme-Q10-hydroquinone,
en utilisant l'oxygène, le bioxyde d'azote, les radicaux stables ou l'adduct peroxyde d'hydrogène-urée et les quinones, dérivés de quinones ou la coenzyme-Q10 -hydroquinone en quantité de 0,3 à 10 mol % par rapport au dérivé carbonylé mis en oeuvre.

2. Procédé selon la revendication 1, caractérisé par le fait que l'on utilise, en tant que radical stable de formule générale I, le radical di-tert-butylamine-N-oxyde de formule Ia

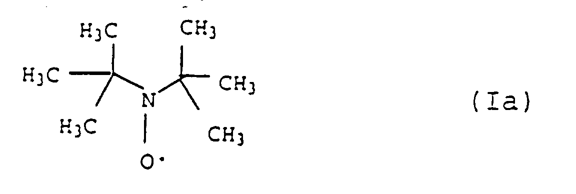

3. Procédé selon la revendication 1, caractérisé par le fait que l'on utilise, en tant que radical stable de formule générale I, un radical 2,2,6,6-tétraméthylpipéridine-N-oxyle de formule Ib 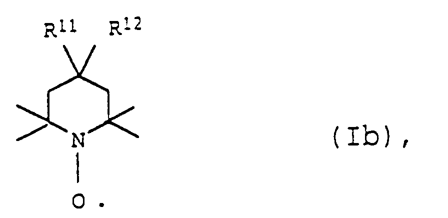 dans laquelle R¹¹ représente l'hydrogène et
R¹² représente l'hydrogène, un groupe hydroxy, alkyloxy, acétoxy, amino, cyano ou carboxyle,
ou bien R¹¹ et R¹² représentent ensemble un groupe oxo.

4. Procédé selon la revendication 1, caractérisé par le fait que l'on utilise, en tant que radical stable de formule générale I, un radical 2,2,5,5-tétraméthylpyrrolidone-N-oxyle de formule Ic 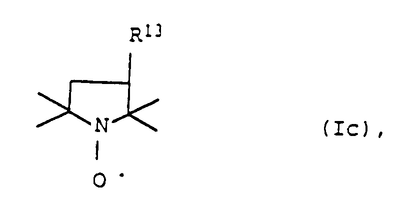 dans laquelle R¹³ représente l'hydrogène ou l'un des groupes mentionnés en référence à R¹² dans la revendication 3.

5. Procédé selon la revendication 1, caractérisé par le fait que l'on utilise, en tant que radical stable de formule générale I, un radical 2,5-dihydro-2,2,5,5-tétraméthyl-1H-pyrrole-N-oxyle de formule Id 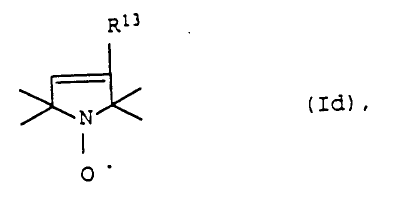 dans laquelle R¹³ a les significations indiquées dans la revendication 4.

6. Procédé selon la revendication 1, caractérisé par le fait que l'on effectue la réaction en présence de quinones ou dérivés de quinones de formules générales X ou XI. 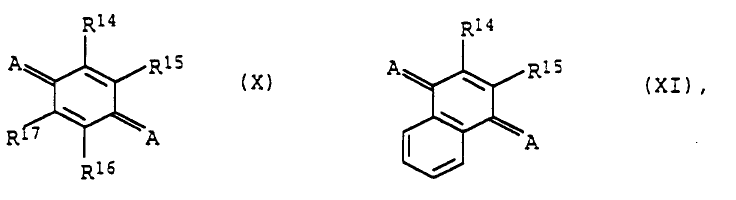 dans lesquelles A représente =O, =NH ou =N-R⁵, R⁵ représentant un groupe alkyle ou bien formant, avec l'un des radicaux R¹ à R⁴ voisins, un groupe alkylène en C2 ou C3 qui peut être substitué par des halogènes, des groupes alkyle en C1-C4 ou alcoxy en C1-C4, et R¹⁴ à R¹⁷ représentent l'hydrogène, des halogènes ou des groupes alkyle, alcényle, cycloalkyle, alcoxy, alcoxycarbonyle, cyano, acyloxy, aryle ou hétéroaryle.

7. Procédé selon la revendication 1, caractérisé par le fait que l'on effectue la réaction dans un hydrocarbure ou hydrocarbure halogéné acyclique, cyclique ou aromatique, dans un alcanol ou dans un solvant polaire aprotonique ou dans un mélange de deux ou plusieurs de ces solvants.

8. Procédé selon la revendication 1, caractérisé par le fait que, pour la préparation d'esters bêta-apo-8'-caroténoïques de formule générale VI 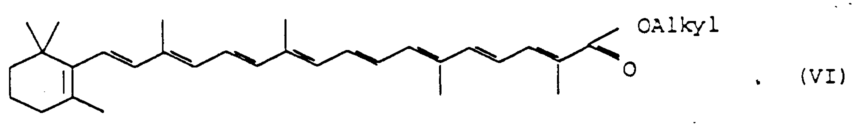 à une teneur en isomères tout-E supérieure à 85 %, on fait réagir le bêta-apo-12'-caroténal (aldéhyde en C25) à une teneur en isomère tout-E supérieure à 85 % en présence d'oxygène ou d'un mélange oxygène-gaz inerte, ou de bioxyde d'azote ou d'un mélange bioxyde d'azote-gaz inerte et/ou en présence du 2,2,6,6-tétraméthylpipéridine-N-oxyle (TEMPO), du 4-hydroxy-2,2,6,6-tétraméthylpipéridine-N-oxyle (4-OH-TEMPO), du 4-oxo-2,2,6,6-tétraméthylpipéridine-N-oxyle, du 3-carboxy-2,2,5,5-tétraméthylpyrrolidine-N-oxyle, du 3-aminocarbonyl-2,2,5,5-tétraméthylpyrrolidine-N-oxyle, du 3-aminocarbonyl-21,5-dihydro-2,2,5,5-tétraméthyl-1H-pyrrole-N-oxyle, du di-tert-butylamine-N-oxyle ou du 2,2-diphényl-1-picrylhydrazale, en tant que radical stable, de la triméthyl-1,4-benzoquinone, de la coenzyme Q10, de la tétrachloro-1,4-benzoquinone, de l'acétate de tocoquinone, de la coenzyme Q0 ou de la 2,2,4-triméthyl-6(H)-quinolone en tant que quinone ou dérivé de quinone, avec un 4-dialkylphosphono-2-méthyl-2-buténoate d'alkyle dans une réaction d'Homer-Emmons.

9. Procédé selon la revendication 1, caractérisé par le fait que pour la préparation d'esters de l'acide neurosporaxanthénoïque de formule générale VII 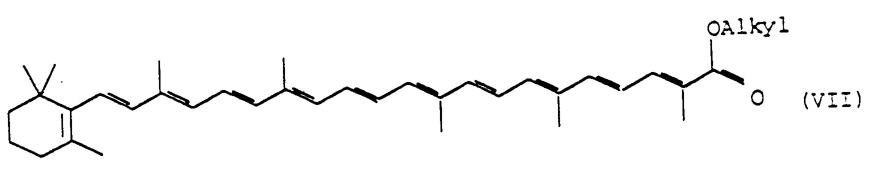 à une teneur en isomère tout-E supérieure à 85 %, on fait réagir le bêta-apo-8'-caroténal à une teneur en isomère tout-E supérieure à 85 %, en présence d'oxygène ou d'un mélange oxygène-gaz inerte ou de bioxyde d'azote ou d'un mélange bioxyde d'azote-gaz inerte et/ou en présence de l'un des radicaux stables mentionnés dans la revendication 8 ou avec l'une des quinones ou l'un des dérivés de quinones mentionnés dans la revendication 8, avec un 4-dialkylphosphono-2-méthyl-2-buténoate d'alkyle dans une réaction d'Homer-Emmons.

10. Procédé selon la revendication 1, caractérisé par le fait que, pour la préparation du citranaxanthène de formule VIII 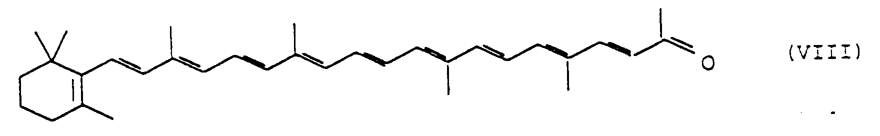 à une teneur en isomère tout-E supérieure à 85 %, on fait réagir le bêta-apo-8'-caroténal à une teneur en isomère tout-E supérieure à 85 %, en présence d'oxygène ou d'un mélange oxygène-gaz inerte ou de bioxyde d'azote ou d'un mélange bioxyde d'azote-gaz inerte, avec l'acétone dans une condensation aldolique.
